# EUROPEAN PATENT SPECIFICATION

(11) **EP 3 851 141 B1**
(45) Date of publication and mention of the grant of the patent: **21.10.2026**
(21) Application number: 20194644.9
(22) Date of filing: 11.05.2018
(51) Int. Cl.: A61B 5/00, A61B 5/021, A61B 5/02, G06N 3/0499, G06N 3/09, A61M 5/172, A61B 5/145, A61B 5/024, A61B 5/026, A61M 5/142

(54) **HIERARCHICAL ADAPTIVE CLOSED-LOOP FLUID RESUSCITATION AND CARDIOVASCULAR DRUG ADMINISTRATION SYSTEM**
HIERARCHISCHE ADAPTIVE FLÜSSIGKEITSWIEDERBELEBUNG MIT GESCHLOSSENEM REGELKREIS UND VERABREICHUNGSSYSTEM FÜR KARDIOVASKULÄRE ARZNEIMITTEL
SYSTÈME DE RÉANIMATION ADAPTATIVE HIÉRARCHIQUE PAR ADMINISTRATION DE FLUIDE EN BOUCLE FERMÉE ET D'ADMINISTRATION DE MÉDICAMENT CARDIOVASCULAIRE

(30) Priority: 12.05.2017 US 201762505232 P
(43) Date of publication of application: 21.07.2021
(62) Document number(s) of the earlier application(s) in accordance with Art. 76 EPC: 18798966.0 / 3 621 676
(73) Proprietor: Autonomous Healthcare, Inc., Hoboken, New Jersey 07030 (US)
(72) Inventor: GHOLAMI, Behnood, Hoboken, NJ New Jersey 07030 (US)
(74) Representative: Jaekel, Robert Sebastian

(56) References cited:
- US-A1- 2005 126 961
- US-A1- 2012 179 135
- US-A1- 2013 218 126

## Description

### BACKGROUND

Reliable and consistent fluid resuscitation (i.e., intravenous administration of fluids) is critical for perioperative and intensive care unit (ICU) fluid management in human and animal populations. The goal of fluid resuscitation is to restore blood volume in the circulatory system to an acceptable level to ensure adequate tissue perfusion. However, large intra-patient and inter-patient variability in physiological parameters, and the effects of different illnesses and medications can result in under-resuscitation and over-resuscitation of patients.
US 2013/0218126 A1 describes a system for closed loop control operation including continuously monitoring a physiological condition and automatic administration of medication regulated by a control unit. US 20050126961 A1 describes a similar system and method for hemofiltration and US 20120179135 A1 a similar system for hemodynamic management including resuscitation and administration of medications.

### SUMMARY OF THE INVENTION

The system according **to the invention is defined in claim 1. Adventageous embodiments** are set out in the dependent **claims.**

### BRIEF DESCRIPTION OF THE FIGURES

**FIG. 1** **PANEL** A illustrates a two-compartment model of fluid exchange in the body for non-burn patients. **FIG. 1** **PANEL B** illustrates a three-compartment model of the microvascular exchange system for burn patients. **FIG.** 1 **PANEL C** illustrates a two-compartment model of cardiovascular drug exchange in the body.
**FIG. 2** **PANEL A** illustrates the overall architecture of a fully automated closed-loop fluid resuscitation or cardiovascular drug administration system. **FIG. 2** **PANEL B** illustrates the overall architecture of a fully automated closed-loop fluid resuscitation and cardiovascular drug administration system.
**FIG. 3** **PANEL A** illustrates the overall architecture of a partially automated (clinical decision support) fluid resuscitation or cardiovascular drug administration system. **FIG. 3** **PANEL B** illustrates the overall architecture of a partially automated (clinical decision support) fluid resuscitation and cardiovascular drug administration system.
**FIG. 4** **PANEL A** illustrates components of a fully automated, closed-loop fluid resuscitation or cardiovascular drug administration system. **FIG. 4** **PANEL B** illustrates components of a fully automated, closed-loop fluid resuscitation and cardiovascular drug administration system.
**FIG. 5** **PANEL** A illustrates components of a partially automated clinical decision support fluid resuscitation or cardiovascular drug administration system. **FIG. 5** **PANEL B** illustrates components of a partially automated clinical decision support fluid resuscitation and cardiovascular drug administration system.
**FIG. 6** illustrates a flow chart of the lower-level adaptive control architecture.
**FIG. 7** depicts stroke volume variation versus time.
**FIG. 8** depicts fluid infusion rates computed by the adaptive control framework.
**FIG. 9** depicts plasma volume in circulation versus time.
**FIG. 10** **depicts** changes in filtered SVV (%) in 2 canine subjects experiencing controlled hemorrhage.
**FIG. 11** depicts changes in infusion rates in 2 canine subjects experiencing controlled hemorrhage.
**FIG. 12** depicts changes in filtered SVV(%) in 3 canine subjects experiencing uncontrolled hemorrhage.
**FIG. 13** depicts changes in infusion rates in 3 canine subjects experiencing uncontrolled hemorrhage.
**FIG. 14** depicts changes in filtered SVV(%) in 2 canine subjects that were hypotensive as a result of administration of sodium nitroprusside (S4-1) and increase in isoflurane (S5-1).
**FIG. 15** depicts changes in infusion rates in 2 canine subjects that were hypotensive as a result of administration of sodium nitroprusside (S4-1) and increase in isoflurane (S5-1).
**FIG. 16** illustrates components of the higher-level controller for the closed-loop fluid resuscitation system.
**FIG. 17** illustrates components of the higher-level controller for the clinical decision support case.
**FIG. 18** depicts mean arterial pressure versus time.
**FIG. 19** depicts cardiovascular drug infusion rates computed by the adaptive control framework.
**FIG. 20** depicts mean arterial pressure versus time.
**FIG. 21** depicts fluid infusion rates computed by the adaptive control framework.
**FIG. 22** depicts stroke volume variation versus time.
**FIG. 23** depicts fluid infusion rates computed by the fluid resuscitation adaptive control framework.
**FIG. 24** depicts mean arterial pressure versus time.
**FIG. 25** depicts vasopressor infusion rates computed by the cardiovascular drug administration adaptive control framework.
**FIG. 26** depicts a fluid resuscitation and cardiovascular drug administration system implemented on hardware.

### DETAILED DESCRIPTION

The system according to the invention is defined in claim 1. Adventageous embodiments are set out in the dependent claims. Reliable and consistent fluid resuscitation (i.e., intravenous administration of fluids) is critical for perioperative and intensive care unit (ICU) fluid management in human and animal populations. Fluid management is required for surgical patients as well as patients suffering from hypovolemia, sepsis, severe sepsis, septic shock, burn, and other conditions. The goal of fluid resuscitation is to restore blood volume in the circulatory system to an acceptable level in order to ensure adequate tissue perfusion (i.e., blood delivery to tissue). However, large intra-patient and inter-patient variability in physiological parameters, and the effects of different illnesses and medications can result in under-resuscitation and over-resuscitation of patients.

Under-resuscitation results in hypovolemia, which can lead to hypoperfusion and organ failure. Over-resuscitation results in fluid overload, which can lead to complications such as pulmonary edema. Fluid overload is associated with higher rates of morbidity and mortality. Restrictive fluid resuscitation protocols reduce the number of mechanical ventilation days and the length of hospital stays.

Cardiovascular drug administration can be used independently to address a clinical condition (e.g., vasopressors are used to increase blood pressure to a clinically acceptable value or inotropic agents are used to change the contractility of the heart). Cardiovascular drugs, such as vasopressors, are administered independent of fluids in critical care for hypotensive patients. Cardiovascular drugs can also be used in combination with fluid resuscitation. For example, to address hypotension and hypovolemia in sepsis and during surgery, a vasopressor and fluid can be administered simultaneously.

The present disclosure describes a reliable and consistent closed-loop fluid resuscitation system, a clinical decision support fluid resuscitation system, a closed-loop cardiovascular drug administration system, a clinical decision support cardiovascular drug administration system, a closed-loop fluid and cardiovascular drug administration system, and a clinical decision support fluid and cardiovascular drug administration system. The clinical decision support fluid resuscitation system, the clinical decision support cardiovascular drug administration system and the clinical decision support fluid and cardiovascular drug administration system do not belong to the present invention but are considered as useful for understanding the invention. The system uses continuous measurements from standard operating room (OR) or ICU hemodynamic monitoring devices or sensors or a built-in or add-on modules to measure such continuous measures to compute the required fluid and/or cardiovascular drug infusion rates for patients receiving continuous infusion. Adaptive control architecture is used to compute the required infusion rates to regulate an endpoint of fluid or drug administration including, but not limited to, static indicators of fluid responsiveness and dynamic indicators of fluid responsiveness for the fluid resuscitation module, and hemodynamic measures for the cardiovascular drug administration module. In some embodiments, the static indicators of fluid responsiveness include mean arterial pressure, central venous pressure, heart rates, cardiac output, stroke volume, cardiac index, and urine output rates of patients. In some embodiments, dynamic indicators of fluid responsiveness include stroke volume variation, pulse pressure variations, systolic pressure variation, dynamic arterial elastance, and pleth variability indices of patients. In some embodiments, hemodynamic measures include mean arterial pressure, central venous pressure, systolic pressure, diastolic pressure, heart rate, cardiac output, cardiac index, systemic vascular resistance, stroke volume, and urine output. The closed-loop fluid resuscitation and/or cardiovascular drug administration system described herein comprises an adaptive controller or two adaptive controllers that use a function approximator, such as a neural network, Fourier functions, or wavelets, to identify the unknown dynamics and physiological parameters of a patient to compute appropriate infusion rates and to regulate the endpoint of fluid resuscitation or cardiovascular drug administration.

The developed fluid resuscitation system can use either crystalloids or colloids during resuscitation and use vasoactive cardiovascular drugs (e.g., vasopressor) or inotropic agents. The fluid infusion rate (e.g., in mL/hour) is highly dependent on a patient needs. In some embodiments, the fluid infusion rate can range from 0 to 3,000 mL/hr or more in humans, and 0 to 40,000 mL/h or more in animals. The cardiovascular drug infusion rate (e.g., in mcg/kg/min) is highly dependent on a patient's needs. In some embodiments, the cardiovascular drug infusion rate (e.g., vasopressor or inotropic agents) can range from 0 mcg/kg/min to 40 mcg/kg/min, or can exceed 40 mcg/kg/min.

### Compartmental modeling for characterizing fluid distribution

The present disclosure models the microvascular exchange system to characterize the distribution of fluids in the body.

A two-compartment dynamic system model is used for all patient populations except for patients with burn injuries. The compartments for two-compartment dynamic system models include circulation (blood) and interstitial tissue. A 4th-order nonlinear state space model representation of the dynamic system can provide a simplified model of the microvascular exchange system for non-burn patients. The states of the two-compartment dynamic system include the volume of fluids and albumin mass in each compartment (a total of four states).

A three-compartment model is used for patients with burn injuries. The states of the three-compartment dynamic system model include circulation (blood), injured tissue, and uninjured tissue. A 6th-order nonlinear state space model representation of the dynamic system can provide a simplified model of the microvascular exchange system for burn patients. The states of the three-compartment dynamic system include volume of fluids and albumin mass in each compartment (a total of six states).

The parameters of the two- and three-compartment models of the microvascular exchange system characterize fluid and mass exchange between different compartments. These parameters are generally unknown and are different from patient to patient.

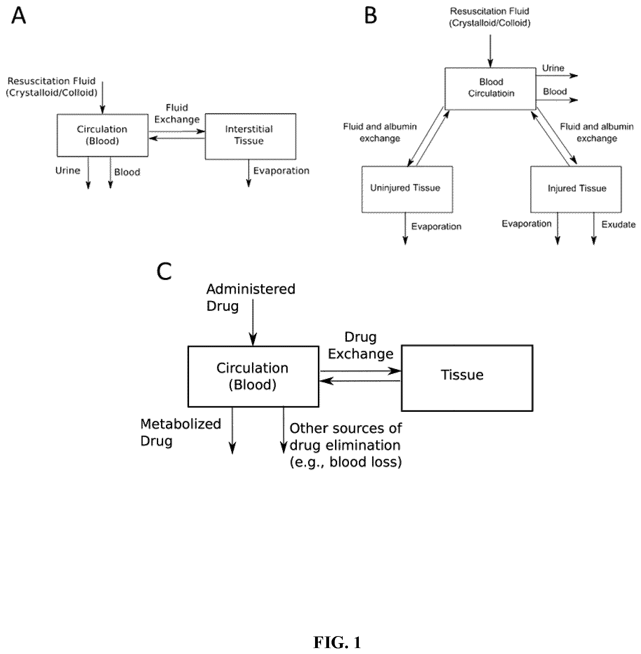

**FIG. 1** **PANEL A** illustrates a two-compartment model of the fluid exchange in the body for non-burn patients. **FIG. 1** **PANEL B** illustrates a three-compartment model of the microvascular exchange system for burn patients.

In some embodiments, a higher number of compartments are used to model the microvascular exchange system of a patient population. In some embodiments, a model dynamic system consists of 2, 3, 4, or 5 compartments. In some embodiments, a 1, 2, 3, or 4-compartment dynamic system model is used for a patient population. In some embodiments, a two-compartment dynamic system model is used for a patient population. In some embodiments, a three-compartment dynamic system model is used for a patient population. In some embodiments, a four-compartment dynamic system model is used for a patient population.

### Compartmental modeling for characterizing cardiovascular drug distribution

The present disclosure models the cardiovascular drug distribution in the body using a compartmental model.

Cardiovascular drug distribution can be modeled using a two-compartment model. The compartments for two-compartment dynamic system models include circulation (blood) and tissue. A 2nd-order nonlinear state space model representation of the dynamic system can provide a simplified model of the cardiovascular drug distribution for patients. The states of the two-compartment dynamic system include the concentration of cardiovascular drug in each compartment.

The parameters of the two-compartment model of the cardiovascular drug distribution characterizes cardiovascular drug mass exchange between different compartments. These parameters are generally unknown and are different from patient to patient. **FIG. 1** **PANEL C** illustrates a two-compartment model of the cardiovascular drug distribution in the body.

In some embodiments, a higher number of compartments are used to model the cardiovascular drug distribution for a patient. In some embodiments, a model dynamic system consists of 2, 3, 4, or 5 compartments. In some embodiments, a 1, 2, 3, or 4-compartment dynamic system model is used for a patient population. In some embodiments, a two-compartment dynamic system model is used for a patient population. In some embodiments, a three-compartment dynamic system model is used for a patient population. In some embodiments, a four-compartment dynamic system model is used for a patient population.

### Process for computing fluid infusion rate and/or cardiovascular drug infusion rate for a closed-loop system or a clinical decision support system

The disclosure provides an adaptive control architecture framework for a closed-loop fluid resuscitation and/or cardiovascular drug administration system. The fluid resuscitation and/or cardiovascular drug administration system of the disclosure receives data from a monitoring system or a set of monitoring systems. In some embodiments, the fluid resuscitation and/or cardiovascular drug administration system receives data from an existing monitoring system or systems. In some embodiments, the fluid resuscitation and/or cardiovascular drug administration system receives data from a built-in monitoring system or systems. In some embodiments, the fluid resuscitation and/or cardiovascular drug administration system receives data from an add-on monitoring system or systems.

The received data comprises one of: blood pressure, heart rate, stroke volume variation, pulse pressure variation, dynamic arterial elastance, pleth variability index, urine output rate, systolic pressure variation, central venous pressure, mean arterial pressure, cardiac output, cardiac index, systolic pressure, diastolic pressure, systemic vascular resistance, or stroke volume. In some embodiments, the received data comprises a combination of blood pressure, heart rate, stroke volume variation, pulse pressure variation, dynamic arterial elastance, pleth variability index, urine output rate or urine output, systolic pressure variation, central venous pressure, mean arterial pressure, cardiac output, cardiac index, systolic pressure, diastolic pressure, systemic vascular resistance, or stroke volume. In some embodiments, a built-in or add-on monitoring system or systems are used and the input data includes one or a combination of an invasive blood pressure signal or a non-invasive pressure signal collected from a patient's arm or leg using a blood pressure cuff.

The disclosed fluid resuscitation and/or cardiovascular drug administration system can transmit data to a receiver or a set of receivers. In some embodiments, the disclosed fluid resuscitation and/or cardiovascular drug administration system can transmit data to an external or built-in infusion pump or infusion pumps, a user, an electronic medical record, or a remote location. In some embodiments, the disclosed fluid resuscitation and/or cardiovascular drug administration system can transmit data to a combination of receivers. In some embodiments, the disclosed fluid resuscitation and/or cardiovascular drug administration system can transmit data to an infusion pump or infusion pumps and a user or users. In some embodiments, the disclosed fluid resuscitation system can transmit data to an infusion pump or infusion pumps, a user or users, and an electric medical record. In some embodiments, the disclosed fluid resuscitation system can transmit data to an infusion pump or infusion pumps, a user or users, an electronic medical record, and a remote location.

The adaptive architecture of the disclosure can be implemented in a fully automated architecture or a partially automated architecture.

**Full automation:** In some embodiments, the adaptive architecture of the disclosure is implemented in a fully automated architecture, wherein the infusion rate of the infusion pump or infusion rates of the infusion pumps are updated automatically by the system using the most recent value of the infusion rate or infusion rates.

**Partial automation (also referred to as clinical decision support):** In some embodiments, the adaptive architecture of the disclosure is implemented in a partially automated architecture. As mentioned above, the clinical decision system related embodiments do not belong to the invention but are considered as useful for understanding the invention. In some embodiments, the framework of the disclosure is used within a clinical decision support context, where recommended infusion rates are displayed to the end-user (clinician) for approval. In some embodiments, the system can request for approval before changing the infusion rate of the pump or pumps. In some embodiments, the end-user can use recommended infusion rate changes and manually change the infusion rate on the pump or pumps based on his/her clinical judgment and the recommendation provided by the clinical decision support system. In some embodiments, the end-user can enter whether the recommended infusion rate was accepted or rejected. In some embodiments, the end-user can enter a new manually changed infusion rate or infusion rates. In some embodiments, the system can automatically change the infusion rate of the pump or infusion rates of the pump after the user approves or modifies the recommended infusion rate or infusion rates.

### Architecture of the disclosure

The present disclosure is able to determine suitable infusion rates based on an input from patient-monitoring devices, built-in monitoring devices, add-on monitoring modules, or sensors. Infusion rates can be adjusted based on the input from patient-monitoring devices, built-in monitoring devices, add-on monitoring modules, or sensors. The adaptive control framework of the disclosure does not require any patient-specific information (e.g., age, gender, weight, diagnosis). Furthermore, the framework does not require an accurate model of the patient dynamics and the patient-specific physiological parameters.

The present disclosure describes a closed-loop or clinical decision support fluid resuscitation and/or cardiovascular drug administration system predicated on hierarchical adaptive control architecture. In some embodiments, the hierarchical control architecture is composed of one or two lower-level adaptive controllers and a higher-level, logic-based controller. In some embodiments, the higher-level, logic-based controller is a rule-based expert system.

If the system is used for fluid resuscitation, the hierarchical control architecture is composed of an adaptive controller fluid module and a higher-level, logic-based controller. If the system is used for only cardiovascular drug administration, the hierarchical control architecture is composed of an adaptive controller cardiovascular drug module and a higher-level, logic-based controller. If the system is used for fluid resuscitation and cardiovascular drug administration, the hierarchical control architecture is composed of an adaptive controller fluid module, an adaptive controller cardiovascular drug module, and a higher-level, logic-based controller.

The lower-level controller focused on fluid resuscitation uses an adaptive control framework to regulate a measure of fluid responsiveness to a desired value by adjusting the fluid infusion rate. In some embodiments, the lower-level controller can regulate mean arterial pressure, systolic pressure, diastolic pressure or a measure of fluid responsiveness including stroke volume variation, pleth variability index, pulse pressure variation, dynamic arterial elastance, central venous pressure, urine output rate or urinte output, or systolic pressure variation. While the goal of this lower-level controller is to regulate a measure of fluid responsiveness to a desired value, the controller may achieve a measurement that is close to the desired value (with some error). Lower-level controller design parameters can be changed to adjust the value of this error.

The lower-level controller focused on cardiovascular drug administration uses an adaptive control framework to regulate a hemodynamic measure to a desired value by adjusting the cardiovascular drug infusion rate. In some embodiments, the lower-level controller can regulate mean arterial pressure, systolic pressure, diastolic pressure, heart rate, cardiac output, stroke volume, systemic vascular resistance, or cardiac index. In some embodiments the administered cardiovascular drug is a vasopressor and the lower-level controller can regulate mean arterial pressure, systolic pressure, systemic vascular resistance, or diastolic pressure. In some embodiments, the administered cardiovascular drug is an inotropic agent and the lower-level controller can regulate cardiac output, cardiac index, mean arterial pressure, systemic vascular resistance, or heart rate. In some embodiments, the administered cardiovascular drug is a chronotropic agent, and the lower-level controller can regulate heart rate or cardiac output. While the goal of this lower-level controller is to regulate a hemodynamic measure to a desired value, the controller may achieve a measurement that is close to the desired value (with some error). Lower-level controller design parameters can be changed to adjust the value of this error.

The role of the higher-level, logic-based controller is different depending on whether the system is used to fully automate or partially automate fluid resuscitation and/or cardiovascular drug administration. The higher-level, logic-based controller monitors the performance of the lower-level adaptive controller(s) and the patient's response to therapy. In case of fluid management, a measure of fluid responsiveness or tissue perfusion is monitored (e.g., mean arterial pressure, stroke volume variation, pulse pressure variation, systolic pressure variation, dynamic arterial elastance, pleth variability index etc.) and in case of cardiovascular drug administration, a measure of hemodynamic function is monitored (e.g., mean arterial pressure, heart rate etc.). If certain performance criteria are violated, then the higher-level controller can "disengage" the lower-level controller(s) (if the system is used for full automation of fluid resuscitation) or the higher-level controller will stop providing suggested infusion rates and will alert the end-user (if the system is used for partial automation of fluid resuscitation). Time stamps, infusion rates and measurement data can be sent to an internal or external database by the higher-level controller for archiving purposes.

The higher-level controller can also determine the timing of engaging each lower-level controller. In some embodiments, the higher-level controller engages the fluid resuscitation module first, and if some performance criteria is not met after a period of time, the drug administration module is also engaged. The higher-level controller (in clinical decision support) can also determine the when to notify the user. In some embodiments, the higher-level controller notifies the user only if the difference between the newly computed infusion rate by the lower-level controller and the last user-approved infusion rate is higher than some threshold.

In some embodiments, in a partial automation application, the higher-level controller can use end-user response (to accept or reject the suggested infusion rate) to update the internal state of the lower-level controller. In addition, in both full automation and partial automation, the higher-level controller can change the internal states of the lower-level controller(s) if the computed infusion rate is out of a "safe" range defined by the end-user. In some embodiments, if the infusion rate computed by the lower-level controller exceeds the maximum allowable infusion rate then the higher-level controller resets the internal parameters and variables of the controller to preset default values.

The higher-level controller can also provide recommendation to the user to engage the cardiovascular drug administration module if a patient's hemodynamic variable of interest is not improved after a certain period of time after fluid resuscitation. In some embodiments, the higher-level controller asks the user to engage the fluid resuscitation module first, and if some performance criteria is not met after a period of time, asks the user to engage the drug administration module. In some embodiments, the higher-level controller asks the user to engage the cardiovascular drug administration module first, and if some performance criteria is not met after a period of time, asks the user to engage the fluid resuscitation module.

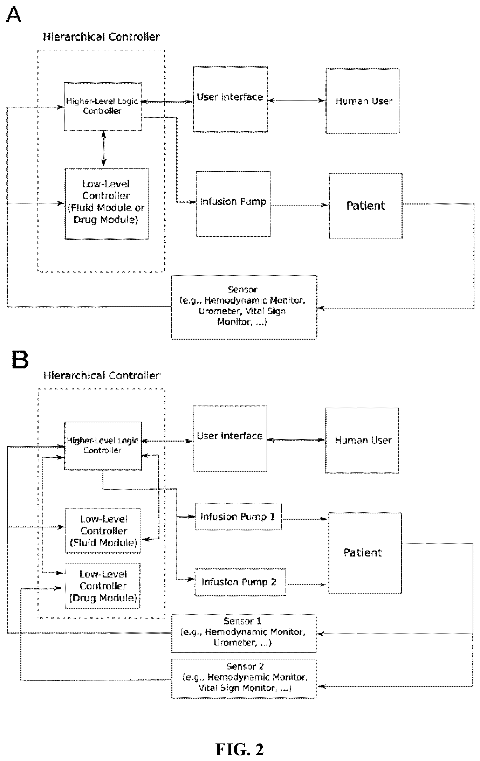

**FIG.** 2 **PANEL** A illustrates the overall architecture of a fully automated closed-loop fluid resuscitation system or a fully automated closed-loop cardiovascular drug administration system of the disclosure. A sensor (e.g., hemodynamic monitor) sends data to the lower-level controller and the higher-level controller. The higher-level controller monitors the performance of the lower-level controller and the response of the patient to fluids or cardiovascular drugs by monitoring measurements from the sensor, internal state of the lower-level controller, and infusion rate computed by the lower-level controller (which is sent to the infusion pump by the higher-level controller). The lower-level controller can send or receive data from the higher-level logic controller.

The higher-level controller can send or receive data from the lower-level controller. The human user (clinician) can interact with the closed-loop system through a user interface to set a target value for the measurement (e.g., set target stroke volume variation of 13% or set mean arterial pressure of 65mmHg), set the range of "safe" infusion rates (e.g., between 0 and 3,000 mL/hr for fluids or 0 and 0.5 mcg/kg/min for a vasopressor), start and stop the system, or set a backup infusion rate in case of loss of sensor signal (e.g., 1,000 mL/hr for fluid or 0.2 mcg/kg/min for vasopressor). The lower-level controller processes a patient's data received from a sensor or a hemodynamic monitoring device (external or built-in), and sends computed infusion rates to the higher-level controller. The higher-level controller ensures that the infusion rate meets all requirements (e.g., it is in the safe range) and if so sends a command to an infusion pump (external or built-in), which administers fluids or cardiovascular drugs to the patient.

**FIG. 2** **PANEL B** illustrates the overall architecture of a fully automated closed-loop fluid resuscitation system and cardiovascular drug administration system of the disclosure. In this system, the closed-loop system provides both fluid and cardiovascular drug simultaneously. One or two sensors (e.g., hemodynamic monitor and vital sign monitor) send data to the lower-level controller fluid module and lower-level controller cardiovascular drug module and the higher-level controller. The higher-level controller monitors the performance of the lower-level controllers and the response of the patient to fluid and cardiovascular drug by monitoring measurements from the sensors, internal state of the lower-level controllers, and infusion rates computed by the lower-level controllers (which are sent to two different infusion pumps by the higher-level controller: a fluid infusion pump and a cardiovascular drug infusion pump). Lower-level controllers can send or receive data from the higher-level logic controller.

The higher-level controller can send or receive data from the lower-level controllers. The human user (clinician) can interact with the closed-loop system through a user interface to set a target value for the measurement or measurements (e.g., set target stroke volume variation of 13% and mean arterial pressure of 65mmHg), set the range of "safe" infusion rates (e.g., between 0 and 3,000 mL/hr for fluids and 0 and 0.5 mcg/kg/min for a vasopressor), start and stop the system, or set a backup infusion rate in case of loss of sensor signal (e.g., 1,000 mL/hr for fluid and 0.2 mcg/kg/min for vasopressor). The lower-level controllers process patient's data received from one or more sensors or a hemodynamic monitoring devices (external or built-in), and sends computed infusion rates to the higher-level controller. The higher-level controller ensures that the infusion rate meets all requirements (e.g., they are in the safe range) and if so sends a commands to infusion pumps (external or built-in), which administer fluids and cardiovascular drugs to the patient. Data measured from sensors used by the lower-level fluid module and lower-level cardiovascular drug administration module may be the same (e.g., mean arterial pressure for both modules) or different (e.g., stroke volume variation for fluid module and mean arterial pressure for cardiovascular drug module).

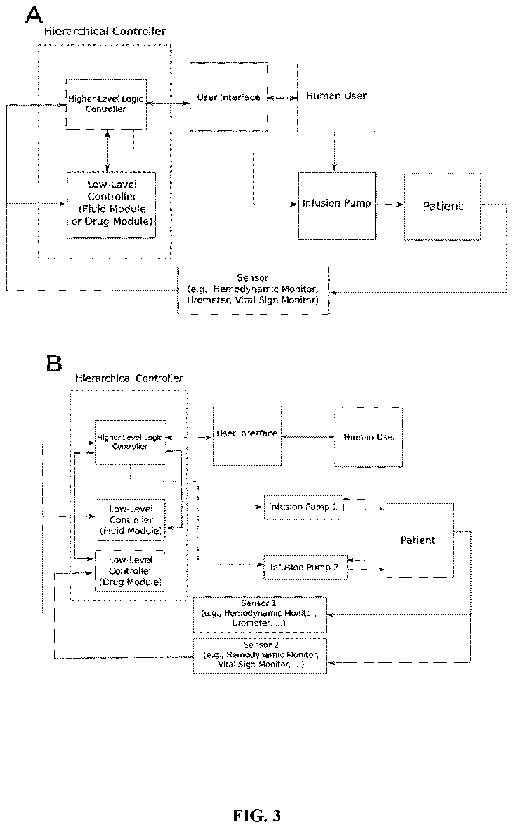

**FIG.** 3 **PANEL** A illustrates the overall architecture of a partially automated (clinical decision support) fluid resuscitation or cardiovascular drug administration system of the disclosure. A monitoring device or sensor (e.g., hemodynamic monitor, vital sign monitor, urometer, etc.) sends data to the lower-level controller and the higher-level controller. The higher-level controller monitors the performance of the lower-level controller and the response of the patient to fluids or cardiovascular drugs by monitoring measurements from the sensor, internal state of the lower-level controller, and infusion rate computed by the lower-level controller and the action taken by the human user (clinician). The lower-level controller can send or receive data from the higher-level logic controller. The higher-level controller can send or receive data from the lower-level controller.

The human user can interact with the partially automated (clinical decision support) system through a user interface to set a target value for the measurement (e.g., set target stroke volume variation of 13% or set target mean arterial pressure of 65mmHg), set the range of "safe" infusion rates (e.g., between 0 and 3,000 mL/hr for fluid or 0 to 0.5 mcg/kg/min for vasopressor), and start and stop the system. The lower-level controller can process a patient's data received from a sensor or a hemodynamic monitoring device (external or built-in), and send recommended infusion rates to the user interface to be displayed. The human user can then either accept or change the recommended rate to an acceptable value. The human user can then manually change the infusion rate on the pump (if pump is not built-in or a pump that is not connected to the system by wire or wireless connection) or instruct the system to change the infusion rate (for built-in or a pump that is connected to the system by wire or wireless connection).

**FIG.** 3 **PANEL B** illustrates the overall architecture of a partially automated (clinical decision support) fluid resuscitation and cardiovascular drug administration system of the disclosure. In this architecture fluid and cardiovascular drug is administered simultaneously. A monitoring deivce or sensor or two monitoring devices and sensors (e.g., hemodynamic monitor, vital sign monitor, urometer) sends data to the lower-level controller fluid module and a lower-level controller cardiovascular drug module and the higher-level controller. The higher-level controller monitors the performance of the lower-level controllers and the response of the patient to fluids and cardiovascular drugs by monitoring measurements from the sensor or sensors, internal state of the lower-level controllers, and infusion rate computed by the lower-level controllers and the action taken by the human user (clinician). Lower-level controllers can send or receive data from the higher-level logic controller. The higher-level controller can send or receive data from the lower-level controllers.

The human user can interact with the partially automated (clinical decision support) system through a user interface to set a target value for the measurement or measurements (e.g., set target stroke volume variation of 13% and set target mean arterial pressure of 65mmHg), set the range of "safe" infusion rates (e.g., between 0 and 3,000 mL/hr for fluid and 0 to 0.5 mcg/kg/min for vasopressor), and start and stop the system. Lower-level controllers can process patient's data received from one or more sensors or hemodynamic monitoring devices (external or built-in), and send recommended infusion rates to the user interface to be displayed. The human user can then either accept or change the recommended rate to an acceptable value. The human user can then manually change the infusion rate on the pump, if the pump is not built-in or a pump is not connected to the system by wire or wireless connection. The human user can also manually instruct the system to change the infusion rate for a built-in pump or a pump that is connected to the system by wire or wireless connection.

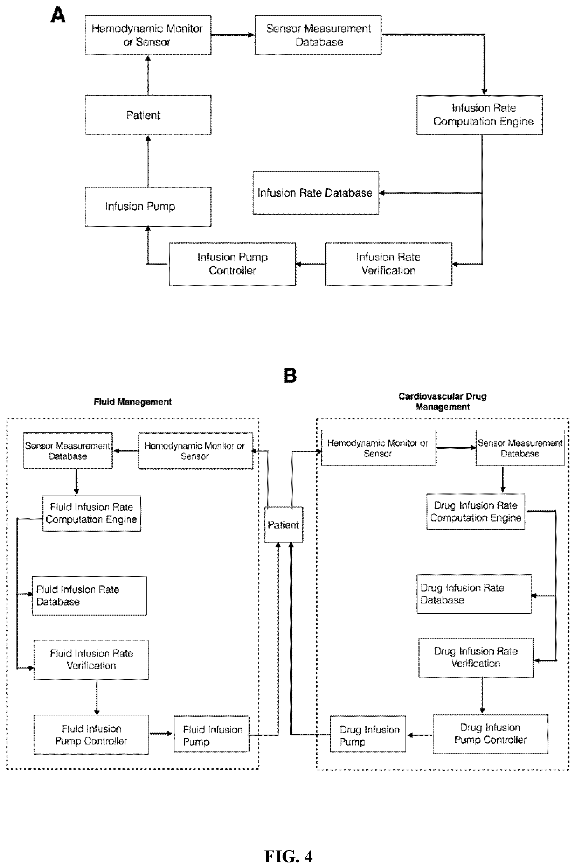

**FIG. 4** **PANEL A** illustrates components of a fully automated, closed-loop fluid resuscitation or cardiovascular drug administration system of the disclosure. A hemodynamic monitor or sensor sends a patient's data to a sensor measurement database. An infusion rate computation engine, which is embedded in the lower-level controller, retrieves sensor measurements and computes infusion rates. The infusion rates (and the corresponding sensor measurements) are communicated with an infusion rate database. The infusion rate computation engine can send data to the infusion rate database and an infusion rate verification system. The infusion rate verification system, which is embedded in the higher-level logic-based controller, ensures the computed rates meet the requirements and if acceptable sends the newly computed infusion rate to the infusion pump controller. The infusion pump controller then automatically changes the infusion rate of the infusion pump to administer an amount of fluid or cardiovascular drug based on commands received by the infusion pump controller.

**FIG. 4** **PANEL B** illustrates components of a fully automated, closed-loop fluid resuscitation and cardiovascular drug administration system of the disclosure. The overall system is composed of two subsystems: a subsystem for fluid management and a subsystem for cardiovascular drug management. In each subsystem, a hemodynamic monitor or sensor sends a patient's data to a sensor measurement database. An infusion rate computation engine, which is embedded in the lower-level controller, retrieves sensor measurements and computes infusion rates. The infusion rates and the corresponding sensor measurements are communicated with an infusion rate database. The infusion rate computation engine can send data to the infusion rate database and an infusion rate verification system. The infusion rate verification system, which is embedded in the higher-level logic-based controller, ensures the computed rates meet the requirements and if acceptable sends the newly computed infusion rate to the infusion pump controller. The infusion pump controller then automatically changes the infusion rate of the infusion pump to administer an amount of fluid or cardiovascular drug based on commands received by the infusion pump controller.

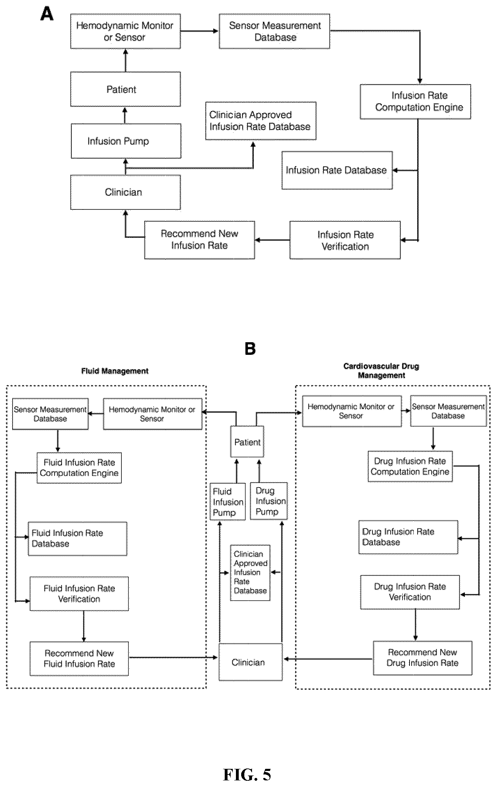

**FIG. 5** **PANEL A** illustrates components of a partially-automated clinical decision support fluid resuscitation or cardiovascular drug administration system of the disclosure. A hemodynamic monitor or sensor sends a patient's data to a sensor measurement database. An infusion rate computation engine, which is embedded in the lower-level controller, retrieves sensor measurements and computes infusion rates. The infusion rates and the corresponding sensor measurements are communicated with an infusion rate database. The infusion rate computation engine sends data to the infusion rate verification system, which is embedded in the higher-level logic-based controller. The infusion rate verification system ensures the computed rates meet the requirements including the requirement to notify the user of significant changes in infusion rate and if acceptable notifies the clinician using a user interface. The recommended new infusion rate is presented to the clinician, who either approves the recommended infusion rate or requests a modification of the rate based on the clinician's qualitative judgement. The approved or modified infusion rate is sent to a database archiving clinician approved infusion rates. The clinician administers the approved or modified fluid or cardiovascular drug by either changing the infusion rate manually, if a pump is not built-in or the pump is not connected to the system by wire or wireless connection. The clinician can also instruct the system to change the infusion rate to the approved value if a pump is built-in or a pump is connected to the system by wire or wireless connection.

**FIG. 5** **PANEL B** illustrates components of a partially-automated clinical decision support fluid resuscitation and cardiovascular drug administration system of the disclosure. The overall system is composed of two subsystems: a subsystem for fluid management and a subsystem for cardiovascular drug management. In each subsystem, a hemodynamic monitor or sensor sends a patient's data to a sensor measurement database. An infusion rate computation engine retrieves sensor measurements and computes infusion rates. The infusion rates and the corresponding sensor measurements are communicated with an infusion rate database. The infusion rate computation engine, which is embedded in the lower-level controller, can send data to the infusion rate verification system. The infusion rate verification system, which is embedded in the higher-level logic-based controller, ensures the computed rates meet the requirements including the requirement to notify the user of significant changes in infusion rate and if acceptable notifies the clinician using a user interface. The recommended new infusion rates are presented to the clinician, who either approves the recommended infusion rates or requests a modification of the rates based on the clinician's qualitative judgement. The approved or modified infusion rates are sent to a database archiving clinician approved infusion rates. The clinician administers the approved or modified fluid and cardiovascular drug by changing the infusion rates manually if a pump is not built-in or a pump that is not connected to the system by wire or wireless connection. The clinician can also administer the approved or modified fluid and cardiovascular drug by instructing the system to change the infusion rates to the approved value if a pump is built-in or a pump is connected to the system by wire or wireless connection.

### EXAMPLES

EXAMPLE 1: Computing fluid or drug infusion rates using lower-level adaptive control architecture.

The present disclosure describes a process of computing the fluid or cardiovascular drug infusion rate using lower-level adaptive control architecture. The lower-level adaptive control architecture is applied to a problem involving only fluid administration, only cardiovascular administration, or fluid and cardiovascular drug administration. In the case of a combined fluid and cardiovascular drug administration, two lower-level adaptive controllers running in parallel are implemented to compute infusion rates for fluid and cardiovascular drug. The fluid or cardiovascular drug infusion rate is computed using lower-level adaptive control architecture through the following steps:
1) Select a sensor that can measure an endpoint for fluid or cardiovascular drug administration. For fluid administration, the endpoint includes variables such as stroke volume variation, pulse pressure variation, mean arterial pressure, dynamic arterial elastance, urine output rate, pleth variability index, dynamic arterial elastance, central venous pressure, systolic pressure, diastolic pressure, or systolic pressure variation. For cardiovascular drug administration, the endpoint includes variables such as mean arterial pressure, cardiac output, cardiac index, heart rate, systolic pressure, diastolic pressure, systemic vascular resistance, or central venous pressure. The values of the sensor are recorded and smoothed using window averaging or other noise reduction techniques. The measurements could be performed invasively or non-invasively.
2) The controller architecture assumes that a patient is modeled by an augmented dynamical system model consisting of a 2-, 3-, or n-compartment model (characterizing fluid or cardiovascular drug distribution), as well as a fictitious state. The fictitious state follows the same trend as of the fluid volume in circulation (in the case of fluid management) or cardiovascular drug mass in circulation (in the case of cardiovascular drug management) with some time lag.
3) A dynamic observer (estimator) is used to estimate deviation of the fictitious state and the fluid volume in circulation (in the case of fluid management) or cardiovascular drug mass in circulation (in the case of cardiovascular drug management) from steady state (equilibrium) values.
4) A function approximator (e.g., neural network or wavelets etc.) is used to approximate the unknown dynamics of the patient and the parameters of the patient, which are modeled by an augmented dynamical system.
5) The function approximator weights (or coefficients), sensor measurement values, and infusion rate computed by the adaptive controller at time *t* - *Δt* are used to compute a new infusion rate. Alternatively, the function approximator weights (or coefficients), sensor measurement values, and infusion rate computed by the adaptive controller at times *t-Δt* and *t-2Δt* are used to compute a new infusion rate.
6) The new infusion rate is sent to higher level controller for further processing.
7) The function approximator weights (or coefficients) are updated using estimates provided by the dynamic observer, sensor measurement values, and the infusion rate computed by the adaptive controller at time *t* - *Δt.* Alternatively, the function approximator weights are updated using estimates provided by the dynamic observer, sensor measurement values, and the infusion rate computed by the adaptive controller at times *t* - Δ*t* and *t - 2Δt.*
8) A delay of T seconds/minutes (e.g., 1 second, 10 seconds, 1 minute, 2 minutes, etc.) is introduced.
9) Step 3) is repeated to close the loop.

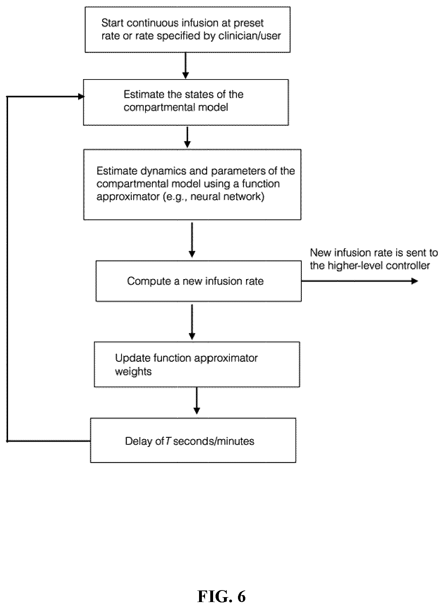

**FIG. 6** details a flow chart of the lower-level adaptive control architecture of the disclosure outlining steps 1) - 9).

**EXAMPLE 2:** Function approximation for modeling unknown dynamics and patient parameters for fluid distribution.

A two-compartment model is used to build the closed-loop fluid resuscitation architecture. The same approach is applied for compartmental models with 3 or more compartments.

A mass balance equation for a two-compartment dynamical system model is given by: ${\dot{v}}_{c} \left(t\right) = u \left(t\right) + {J}_{L} \left(t\right) - {J}_{t} \left(t\right) - {J}_{urine} \left(t\right) - {J}_{blood} \left(t\right) , t \geq 0$ ${\dot{a}}_{c} \left(t\right) = {Q}_{L} \left(t\right) - {Q}_{t} \left(t\right) - {a}_{blood} \left(t\right) ,$ ${\dot{v}}_{t} \left(t\right) = {J}_{t} \left(t\right) - {J}_{L} \left(t\right) - {J}_{evaporation} \left(t\right) ,$ ${\dot{a}}_{t} \left(t\right) = - {Q}_{L} \left(t\right) + {Q}_{t} \left(t\right) ,$ where *u(t)* is the rate of fluid infusion, *J*_{L}(*t*) is the rate of volume transfer from interstitial tissue to circulation, *J*ₜ *(t)* is the rate of volume transfer from circulation to interstitial tissue, *J*ᵤᵣᵢₙₑ(*t*), *J*_{blood}(*t*), and *J*ₑᵥₐₚₒᵣₐₜᵢₒₙ(*t*) denote loss of fluid volume due to urine production, hemorrhage, or evaporation (and other types of insensible water loss), respectively. Furthermore, *a*_{blood}(*t*) denotes the rate of loss of protein (albumin) mass from hemorrhage, *Qₜ*(*t*) is the rate of albumin mass transfer from circulation to interstitial tissue, and *Q*_{L}(*t*) is the rate of albumin mass transfer from interstitial tissue to circulation.

The above equation can be rewritten in state space form, namely, ${\dot{v}}_{c} \left(t\right) = {f}_{1} \left(v\left(t\right)\right) + g \left(v\left(t\right)\right) u \left(t\right) , {v}_{c} \left(0\right) = {v}_{c , 0} , t \geq 0$ (1) ${\dot{a}}_{c} \left(t\right) = {f}_{2} \left(v\left(t\right)\right) , {a}_{c} \left(0\right) = {a}_{c , 0}$ (2) ${\dot{v}}_{t} \left(t\right) = {f}_{3} \left(v\left(t\right)\right) , {v}_{t} \left(0\right) = {v}_{t , 0}$ (3) ${\dot{a}}_{t} \left(t\right) = {f}_{4} \left(v\left(t\right)\right) , {a}_{t} \left(0\right) = {a}_{t , 0}$ (4) where for *t* ≥ *0, v(t)* = [*v_{c}*(*t*)*, a*_{c}(*t*), *v*ₜ(*t*), *a*ₜ(*t*)]*^{T}*, and *v*_{c}(*t*)*, a*_{c}(*t*)*, vt(t),* and *a*ₜ(*t*) denote fluid volume in circulation, albumin mass in circulation, fluid volume in interstitial tissue, and albumin mass in interstitial tissue, respectively. In addition, *f*₁(*v*) and *f*₃(*v*) denote functions characterizing the rate of change of fluid in circulation and tissue compartments, respectively, and *f*₂(*v*) and *f*₄(*v*) denote functions characterizing the rate of change of albumin in circulation and tissue compartments, respectively, *g(v (t))* characterizes the effect of fluid infusion on the compartmental model. Note that variables indicating exchange of volume or mass with the outside environment including , *J*ᵤᵣᵢₙₑ(*t*)*, J*_{blood}(t), *J*ₑᵥₐₚₒᵣₐₜᵢₒₙ(*t*), and *a*_{blood}(*t*) have been incorporated into functions *f*₁(*v*), *f*₂(*v*), and *f*₃(*v*). Note that *v*_{*c*,0}, *a_{c,0}, v*_{t,0}, and *a*_{t,0} denote volume and albumin mass in circulation and interstitial tissue at t=0, respectively. Also note that the functions *f*₁(*v*), *f*₂(*v*)*, f*₃(*v*)*, f*₄(*v*)*,* and g(v) are generally unknown for each individual patient.

The original two-compartment model is then modified to introduce a certain structure in the dynamics. A fictitious state, *x*_{f}(*t*), is added such that the fictitious state follows the same trend as the volume of fluid in circulation, *v*_{c}(*t*), with some time lag. The dynamics of the fictitious state is given by the linear differential equation ${\dot{x}}_{f} \left(t\right) = {c}_{1} {v}_{c} \left(t\right) + {c}_{2} {x}_{f} \left(t\right) , {x}_{f} \left(0\right) = 0 , t \geq 0 ,$ (5) where *c*₁, *c*₂ ∈ R are design parameter. Next, error variables are defined that quantify the deviation of each variable from its equilibrium state (steady state value). The error variables are defined by equations (6) - (10): ${e}_{f} \left(t\right) = {x}_{f} \left(t\right) - {x}_{f , e}$ (6) ${e}_{v , c} \left(t\right) = {v}_{c} \left(t\right) - {v}_{c , e}$ (7) ${e}_{a , c} \left(t\right) = {a}_{c} \left(t\right) - {a}_{c , e}$ (8) ${e}_{v , t} \left(t\right) = {v}_{t} \left(t\right) - {v}_{t , e}$ (9) ${e}_{a , t} \left(t\right) = {a}_{t} \left(t\right) - {a}_{t , e}$ (10) where *e*_{f}(*t*) denotes the deviation of the fictitious state from fictitious state equilibrium value *x*_{f,e}, *e*_{v,c}(*t*) denotes the deviation of fluid volume in circulation from its equilibrium value *v*_{c,e}, *e_{a,c}*(*t*) denotes the deviation of albumin mass from its equilibrium value *a*_{c*,*e}*, e*_{v*,*t}(*t*) denotes the deviation of fluid volume in tissue from its equilibrium value *v*_{t,e}, and *e*_{a*,t*}(*t*) denotes the deviation of albumin mass from its equilibrium value *a*_{t,e}.

It follows from (5) that ${υ}_{c , e} = - \frac{{c}_{2}}{{c}_{1}} {x}_{f , e} .$

Hence, (1) - (5) can be rewritten as ${\dot{e}}_{f} \left(t\right) = {c}_{1} {e}_{v , c} \left(t\right) + {c}_{2} {e}_{f} \left(t\right)$ (11) ${\dot{e}}_{v , c} \left(t\right) = {\hat{f}}_{1} \left({e}_{v}\left(t\right)\right) + \hat{g} \left({e}_{v}\left(t\right)\right) u \left(t\right) , {e}_{v , c} \left(0\right) = {e}_{v , c , 0} , t \geq 0$ (12) ${\dot{e}}_{a , c} \left(t\right) = {\hat{f}}_{2} \left({e}_{v}\left(t\right)\right) , {e}_{a , c} \left(0\right) = {e}_{a , 0} ,$ (13) ${\dot{e}}_{v , t} \left(t\right) = {\hat{f}}_{3} \left({e}_{v}\left(t\right)\right) , {e}_{v , t} \left(0\right) = {e}_{v , t , 0} ,$ (14) ${\dot{e}}_{a , t} \left(t\right) = {\hat{f}}_{4} \left({e}_{v}\left(t\right)\right) , {e}_{a , t} \left(0\right) = {e}_{a , t , 0} ,$ (15) where for *t* ≥ *0, e*ᵥ(*t*) *=* [*e*_{v,c}(*t*)*, e*_{a,c}(*t*)*, e*_{v,t}(*t*)*, e*_{a,t}(*t*)] , and *f*₁(*v*(*t*))*, f*₂(*v*(*t*))*, f*₃(*v*(*t*))*, f*₄(*v*(*t*)), *g(v(t))* are rewritten as *f̂*₁ (*e*ᵥ*(t)), f̂₂* (*e*ᵥ(*t*))*, f̂*₃ (*e*ᵥ(*t*))*, f̂*₄(*e*ᵥ(*t*))*, ĝ*(*eᵥ*(*t*))*,* respectively, after performing a change of variables using equations (6) - (10). Parameters α₁ and α₂ are then introduced, where α₁, *α*₂ E R, and equation (12) is rewritten as ${\dot{e}}_{v , c} \left(t\right) = {α}_{1} {e}_{v , c} \left(t\right) + {α}_{2} {e}_{f} \left(t\right) + h \left(\overline{ξ}\left(t\right)\right) + \hat{g} \left({e}_{v}\left(t\right)\right) u \left(t\right) , {e}_{v , c} \left(0\right) = {e}_{v , c , 0} , t \geq 0 ,$ where $h \left(\overline{ξ}\left(t\right)\right) = {\hat{f}}_{1} \left({e}_{v}\left(t\right)\right) - {α}_{1} {e}_{v , c} \left(t\right) - {α}_{2} {e}_{f} \left(t\right) ,$ (16) and $\overline{ξ} \left(t\right) = {\left[{e}_{v , c}\left(t\right),{e}_{f}\left(t\right),{e}_{a , c}\left(t\right),{e}_{v , t}\left(t\right),{e}_{a , t}\left(t\right)\right]}^{T} .$

Next, a series of basis functions are used, such as neural networks basis functions (e.g., radial basis functions or sigmoids), wavelets, or Fourier functions, to approximate h(ξ̅) and *ĝ*(ξ̅). Specifically, $h \left(\overline{ξ}\right) = {w}_{p}^{T} \left(t\right) p \left(\overline{ξ}\right) + {ε}_{p}$ $\hat{g} \left(\overline{ξ}\right) = {w}_{q}^{T} \left(t\right) q \left(\overline{ξ}\right) + {ε}_{q}$ (17) where *p*₁(ξ̅)*,* ... *,p_{nₚ}*(*ξ̅*) and *q*₁(*ξ̅*), ... , q_{n_{q}} (ξ̅) are two sets of basis functions, *p*(*ξ̅*) *=* [*p*₁(ξ̅), . . . ,*p_{nₚ}* (ξ̅)]^{T}, *q*(ξ̅) = [*q*₁(*ξ̅*), ...,*q_{n_{q}}*(*ξ̅*)]^{T}, ${w}_{p} \left(t\right) ≜ {\left[{w}_{p , 1}\left(t\right), …,{w}_{p , {n}_{p}}\left(t\right)\right]}^{T}$, and ${w}_{q} \left(t\right) ≜ {\left[{w}_{q , 1}\left(t\right), …,{w}_{q , {n}_{q}}\left(t\right)\right]}^{T}$ are time varying weights (or coefficients) corresponding to the basis functions, and εₚ and ε_{q} are the approximation errors.

The presented framework is general; however, for illustration purposes, sigmoidal neural network basis functions of the form $s \left(\overline{ξ}\right) ≜ \frac{1}{1 + {e}^{σ \left(\overline{ξ}-{\overline{ξ}}_{0}\right)}} , σ , {t}_{0} ∈ ℝ$, can be used. α₁, *α₂, c*₁*,* and *c*₂ can be selected such that $A ≜ \left[\begin{matrix}{c}_{2} & {c}_{1} \\ {α}_{1} & {α}_{2}\end{matrix}\right] ,$ (19) is asymptotically stable (i.e., the real parts of all the eigenvalues *of A* are negative). One specific choice for these parameters are *c*₁ = 100, *c*₂ = -100, *α*₁ = 0, and *α*₂ = -100.

EXAMPLE 3: Function approximation for modeling unknown dynamics and patient parameters for cardiovascular drug distribution.

A two-compartment model is used to build the closed-loop cardiovascular administration architecture disclosed herein. The same approach is applied for compartmental models with 3 or more compartments.

A drug mass balance equation for a two-compartment dynamical system model is given by: ${\dot{d}}_{c} \left(t\right) = u \left(t\right) - {J}_{M} \left(t\right) - {J}_{t} \left(t\right) + {J}_{c} \left(t\right) - {J}_{other} \left(t\right) , t \geq 0$ ${\dot{d}}_{t} \left(t\right) = {J}_{t} \left(t\right) - {J}_{c} \left(t\right) ,$ where *u(t)* is the rate of drug infusion, *J*_{M}(*t*) is the rate of drug mass elimination from circulation as a result of metabolism, *J*ₜ *(t)* is the rate of drug mass transfer from circulation to tissue, *J_{c} (t)* is the rate of drug mass transfer from tissue to circulation, Jₒₜₕₑᵣ(*t*) is the rate of drug elimination from circulation other than metabolism (e.g., as a result of hemorrhage) .

The above equation can be rewritten in state space form, namely, ${\dot{d}}_{c} \left(t\right) = {f}_{1} \left(d\left(t\right)\right) + g \left(d\left(t\right)\right) u \left(t\right) , {d}_{c} \left(0\right) = {d}_{c , 0} , t \geq 0$ (20) ${\dot{d}}_{t} \left(t\right) = {f}_{2} \left(d\left(t\right)\right) , {d}_{t} \left(0\right) = {d}_{t , 0}$ (21) where for *t* ≥ *0, d(t)* = [*d_{c}*(t)*, d*ₜ(*t*)]*^{T},* and *d*_{c}(*t*) and *d*ₜ(*t*) denote cardiovascular drug mass in circulation and tissue, respectively. In addition, *f*₁(*d*) and *f*₂(*d*) denote functions characterizing the rate of change of drug mass in circulation and tissue compartments, respectively, *g(d(t))* characterizes the effect of cardiovascular drug infusion on the compartmental model. Note that variables indicating exchange of mass with the outside environment, that is, *J*ₒₜₕₑᵣ(*t*), and *J*_{M}(t), have been incorporated into function *f*₁(*d*)*,* and *f*₂(*v*). Note that *d*_{c,0}, and *d*_{t,0}, denote drug mass in circulation and tissue at t=0, respectively. Also note that the functions *f*₁(*d*), *f*₂(*d*), and *g(d)* are generally unknown for each individual patient.

The original two-compartment model is then modified to introduce a certain structure in the dynamics. A fictitious state, *x*_{f}(*t*), is added such that the fictitious state follows the same trend as the cardiovascular drug mass in circulation, *d*_{c}(*t*)*,* with some time lag. The dynamics of the fictitious state is given by the linear differential equation ${\dot{x}}_{f} \left(t\right) = {c}_{2} {d}_{c} \left(t\right) + {c}_{1} {x}_{f} \left(t\right) , {x}_{f} \left(0\right) = 0 , t \geq 0 ,$ (22) where *c*₁, *c*₂ ∈ R are design parameters. Next, error variables are defined that quantify the deviation of each variable from its equilibrium state (steady state value). The error variables are defined by equations (23)-(26): ${e}_{f} \left(t\right) = {x}_{f} \left(t\right) - {x}_{f , e}$ (23) ${e}_{d , c} \left(t\right) = {d}_{c} \left(t\right) - {d}_{c , e}$ (24) ${e}_{d , t} \left(t\right) = {d}_{t} \left(t\right) - {d}_{t , e}$ (25) where *e*_{f}(*t*) denotes the deviation of the fictitious state from fictitious state equilibrium value x_{f,e}, *e*_{d*,*c}(*t*) denotes the deviation of cardiovascular drug mass in circulation from its equilibrium value *d*_{c,e}, and *e*_{d,*t*}(*t*) denotes the deviation of cardiovascular drug mass in tissue from its equilibrium value *d*_{t,e}.

It follows from (22) that ${d}_{c , e} = - \frac{{c}_{1}}{{c}_{2}} {x}_{f , e}$*.* Hence, (20) - (22) can be rewritten as ${\dot{e}}_{f} \left(t\right) = {c}_{2} {e}_{d , c} \left(t\right) + {c}_{1} {e}_{f} \left(t\right)$ (26) ${\dot{e}}_{d , c} \left(t\right) = {\hat{f}}_{1} \left({e}_{d}\left(t\right)\right) + \hat{g} \left({e}_{d}\left(t\right)\right) u \left(t\right) , {e}_{d , c} \left(0\right) = {e}_{d , c , 0} , t \geq 0$ (27) ${\dot{e}}_{d , t} \left(t\right) = {\hat{f}}_{2} \left({e}_{d}\left(t\right)\right) , {e}_{d , t} \left(0\right) = {e}_{d , t , 0} ,$ (28) where for *t* ≥ *0, e*_{d}(*t*) = [*e*_{d*,*c}(*t*)*, e*_{d*,*t}(*t*)]*,* and *f*₁(*d*(*t*))*, f*₂(*d*(*t*))*, g(d(t))* are rewritten as *f̂*₁(*e*_{d}(*t*))*, f̂*₂(*e*_{d}(*t*))*, ĝ*(*e*_{d}(*t*))*,* respectively, after performing a change of variables using equations (23) - (26). Parameters α₁ and *α₂* are then introduced, where α₁, *α₂* E R, and equation (12) is rewritten as ${\dot{e}}_{d , c} \left(t\right) = {α}_{1} {e}_{d , c} \left(t\right) + {α}_{2} {e}_{f} \left(t\right) + h \left(\overline{ξ}\left(t\right)\right) + \hat{g} \left({e}_{d}\left(t\right)\right) u \left(t\right) , {e}_{d , c} \left(0\right) = {e}_{d , c , 0} , t \geq 0 ,$ where $h \left(\overline{ξ}\left(t\right)\right) = {\hat{f}}_{1} \left({e}_{d}\left(t\right)\right) - {α}_{1} {e}_{d , c} \left(t\right) - {α}_{2} {e}_{f} \left(t\right) ,$ (29) and $\overline{ξ} \left(t\right) = \left[{e}_{d , c}\left(t\right),{e}_{f}\left(t\right),{e}_{d , t}\left(t\right)\right] .$

Next, a series of basis functions are used, such as neural networks basis functions (e.g., radial basis functions or sigmoids), wavelets, or Fourier functions, to approximate *h*(*ξ̅*) and *ĝ*(*ξ̅*). Specifically, $h \left(\overline{ξ}\right) = {w}_{p}^{T} \left(t\right) p \left(\overline{ξ}\right) + {ε}_{p}$ (30) $\hat{g} \left(\overline{ξ}\right) = {w}_{q}^{T} \left(t\right) q \left(\overline{ξ}\right) + {ε}_{q}$ (31)' where
*p*₁*(*ξ̅)*,* ... *, p_{nₚ}* (ξ̅) and *q*₁(ξ̅)*,* . .. , *q_{n_{q}}* (ξ̅) are two sets of basis functions, *p*(*ξ̅*) *=* [*p*₁(*ξ̅*)*,* . . . , *p_{nₚ}* (ξ̅)]^{T}, *q*(*ξ̅*) = [*q*₁(*ξ̅*), ...,q_{n_{q}}(ξ̅)]^{T}, ${w}_{p} \left(t\right) ≜ {\left[{w}_{p , 1}\left(t\right), …,{w}_{p , {n}_{p}}\left(t\right)\right]}^{T}$, and ${w}_{q} \left(t\right) ≜ {\left[{w}_{q , 1}\left(t\right), …,{w}_{q , {n}_{q}}\left(t\right)\right]}^{T}$ are time varying weights (or coefficients) corresponding to the basis functions, and εₚ and ε_{q} are the approximation errors.

The presented framework is general; however, for illustration purposes, sigmoidal neural network basis functions of the form $s \left(\overline{ξ}\right) ≜ \frac{1}{1 + {e}^{σ \left(\overline{ξ}-{\overline{ξ}}_{0}\right)}} , σ , {t}_{0} ∈ ℝ$, can be used. α₁, *α₂, c*₁*,* and c₂ can be selected such that $A ≜ \left[\begin{matrix}{c}_{1} & {c}_{2} \\ {α}_{1} & {α}_{2}\end{matrix}\right] ,$ (32) is asymptotically stable (i.e., the real parts of all the eigenvalues *of A* are negative). One specific choice for these parameters are *c*₁ = 0.2, *c*₂ = 0.013, α₁ = 0, and α₂ = -0.2.

EXAMPLE 3: Computing the value of continuous infusion.

At each time instant t, the infusion rate of the controller (for fluid or cardiovascular drug) is given by $u \left(t\right) = \left\{\begin{matrix}0 , & \hat{u} \left(t\right) \leq 0 , \\ \hat{u} \left(t\right) , & \hat{u} \left(t\right) > 0 ,\end{matrix}\right.$ where $u \left(t\right) = - \frac{1}{1 + {w}_{q}^{T} \left(t\right) Q \left(z\left(t\right)\right)} {w}_{p}^{T} \left(t\right) P \left(z\left(t\right)\right) ,$ and $z \left(t\right) ≜ {\left[m\left(t-Δt\right),m\left(t-2Δt\right),u\left(t-Δt\right),u\left(t-2Δt\right)\right]}^{T} ,$ $P \left(z\left(t\right)\right) = Q \left(z\left(t\right)\right) ≜ \left[\begin{matrix}\frac{1}{1 + {e}^{- {σ}_{1} \left(m\left(t-Δt\right)-{m}_{target}\right)}} , … , \frac{1}{1 + {e}^{- {σ}_{{n}_{node}} \left(m\left(t-Δt\right)-{m}_{target}\right)}} , \\ \frac{1}{1 + {e}^{- {σ}_{1} \left(m\left(t-2Δt\right)-{m}_{target}\right)}} , … , \frac{1}{1 + {e}^{- {σ}_{{n}_{node}} \left(m\left(t-2Δt\right)-{m}_{target}\right)}} , \\ \frac{1}{1 + {e}^{- {σ}_{1} u \left(t-Δt\right)}} , … , \frac{1}{1 + {e}^{- {σ}_{{n}_{node}} \left(t-Δt\right)}} , \\ \frac{1}{1 + {e}^{- {σ}_{1} u \left(t-2Δt\right)}} , … , \frac{1}{1 + {e}^{- {σ}_{{n}_{node}} u \left(t-2Δt\right)}}\end{matrix}\right] ,$ σ₁, ..., σ_{node}, are sigmoid parameters (e.g., ranging from -100 to 100), *n*_{node} represents the number of nodes of the neural network (e.g., *n*_{node} = 8), and *m(t)* represents the smoothed (denoised) sensor measurement used as an endpoint for fluid or drug administration (e.g., stroke volume variation, urine output rate, mean arterial pressure, central venous pressure, systolic pressure variation, etc. for fluid resuscitation; and mean arterial pressure, heart rate, systolic pressure, diastolic pressure, systemic vascular resistance, central venous pressure, etc. for cardiovascular drug administration) at time *t. u(t)* represents computed infusion rate at time *t.* For example, smoothed (denoised) sensor measurement can be obtained by a moving window average where the mean value of the sensor measurement in a time window (e.g., 2 minutes) is computed and assigned to *m(t).* Sensor values can be preprocessed to drop measurements that appear to be noisy or invalid and only acceptable values are included in the window averaging.

Alternatively, $z \left(t\right) ≜ {\left[m\left(t-Δt\right),u\left(t-Δt\right)\right]}^{T} ,$ $P \left(z\left(t\right)\right) = Q \left(z\left(t\right)\right) ≜ \left[\begin{matrix}\frac{1}{1 + {e}^{- {σ}_{1} \left(m\left(t-Δt\right)-{m}_{target}\right)}} , … , \frac{1}{1 + {e}^{- {σ}_{{n}_{node}} \left(m\left(t-Δt\right)-{m}_{target}\right)}} , \\ \frac{1}{1 + {e}^{- {σ}_{1} u \left(t-Δt\right)}} , … , \frac{1}{1 + {e}^{- {σ}_{{n}_{node} u \left(t-Δt\right)}}}\end{matrix}\right] .$

The update laws for the weights are given by the set of ordinary differential equations ${\dot{w}}_{p} \left(t\right) = {β}_{1} Γ \left({w}_{p}\left(t\right),-P\left(z\left(t\right)\right){x}_{c}^{T}\left(t\right){P}^{˜}{B}_{0}\right) , {w}_{p} \left(0\right) = {w}_{p , 0} , t \geq 0 ,$ ${\dot{w}}_{q} \left(t\right) = {β}_{2} Γ \left({w}_{q}\left(t\right),-Q\left(z\left(t\right)\right)u\left(t\right){x}_{c}^{T}\left(t\right){P}^{˜}{B}_{0}\right) , {w}_{q} \left(0\right) = {w}_{q , 0}$ ${\dot{x}}_{c} \left(t\right) = {Ax}_{c} \left(t\right) + L \left(m\left(t\right)-{m}_{target}-{y}_{c}\left(t\right)\right) , {x}_{c} \left(0\right) = 0 ,$ ${y}_{c} \left(t\right) = {Cx}_{c} \left(t\right) ,$ where *x*_{c}(*t*) = [*x*_{*c,*1}(*t*)*, x*_{c*,*2}(*t*)]*^{T}* represents the estimated values of *e_{f}*(*t*) and *e*_{v*,*c}(*t*) (in the case of fluid resuscitation) or *e*_{f}(*t*) and *e*_{d,c}(*t*) (in the case of cardiovascular drug administration), *β*₁, *β*₂ > 0 denote design parameters (e.g., *β*₁ = 0.02 and *β*₂ = 0.04), and representative values for other parameters are given by L = [-1, 0]^{T}, *B*₀ = [0, 1]^{T}, and C = [-1, 0]^{T}. In addition, *m*_{target} is the desired value for the end point of fluid resuscitation or cardiovascular drug administration (e.g., for fluid resuscitation if the goal is to maintain a stroke volume variation of 13%, then *m*_{target} *=* 13; similarly, for cardiovascular drug administration if the goal is to maintain a mean arterial pressure of 65mmHg, then *m*_{target} *=* 65), and *P̃* satisfies the Lyapunov equation given by: ${\left(A-LC\right)}^{T} {P}^{˜} + {P}^{˜} \left(A-LC\right) + {R}^{˜} = 0 ,$ (33) where *R̃* > 0.

For example, if *R̃* = *I*_{2×2} and the parameter values above are used, then ${P}^{˜} = \left[\begin{matrix}0.4116 & 0.0039 \\ 0.0039 & 2.5\end{matrix}\right]$ for cardiovascular drug administration, and ${P}^{˜} = \left[\begin{matrix}0.0005 & 0.003 \\ 0.003 & 0.008\end{matrix}\right]$ for fluid resuscitation. In addition, the function Γ(*θ*, y) is used to ensure that controller parameters remain bounded. This function is defined as: $Γ \left(θ, y\right) ≜ \left\{\begin{matrix}y , & if f \left(θ\right) < 0 \\ y , & if f \left(θ\right) \geq 0 , and ∇ {f}^{T} y \leq 0 , \\ y - \frac{∇ f}{\left‖∇f\right‖} \left〈\frac{∇ f}{\left‖∇f\right‖}, y\right〉 f \left(θ\right) , & if f \left(θ\right) \geq 0 and ∇ {f}^{T} y > 0 ,\end{matrix}\right.$ where *f*: R" → R is defined as $f \left(θ\right) ≜ \frac{\left({ε}_{θ}+1\right) {θ}^{T} θ - {θ}_{max}^{2}}{{ε}_{θ} {θ}_{max}^{2}} ,$ (34) *θ*ₘₐₓ > 0, *ε*_{θ} *>* 0, ∇(·) represents the gradient operator, and ∥ · ∥ represents the Euclidean norm. For example, *θ*ₘₐₓ =1e6 and ε_{θ} =1e-5.

**EXAMPLE 4:** Computer simulations for fluid resuscitation.

Adaptive control framework is used to simulate fluid resuscitation of a 70kg patient losing blood at a rate of 2ml/kg/min at *t* = 0. The goal is to maintain the stroke volume variation measurements at 15%. A Δ*t* of 0.001 hour (3.6 seconds) is used for the simulations and *β*₁ = 2, *β*₂ = 4, and *n*_{node} = 8. The patient model involved a compartmental model to model fluid distribution and the relationship between volume in circulation and SVV was based on a nonlinear relationship based on experimental results on dogs.

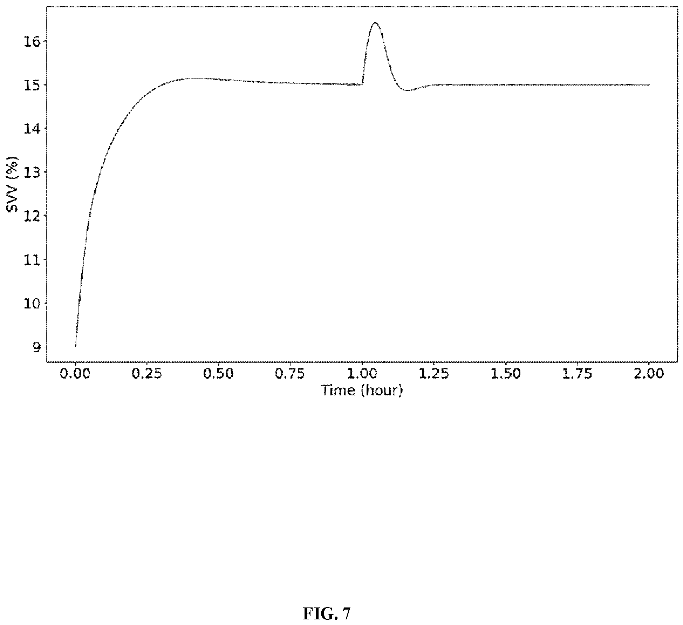

**FIG.** 7 shows stroke volume variation (SVV (%)) versus time. The target stroke volume variation is 15%. The SVV (%) starts at about 9 %, and changes with the introduction of fluid resuscitation. The SVV (%) increases to the target value of 15%. At *t* = 1 hr, the blood loss increases to 3mL/kg/min, and the controller increases the infusion rate to drive stroke volume variation measurements to the target value of 15%.

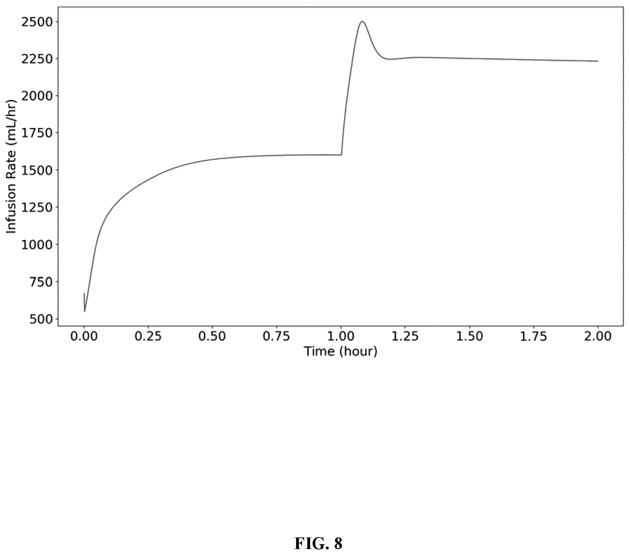

**FIG. 8** shows infusion rates computed by the adaptive control framework. The infusion rate is about 700 mL/h at t=0 (start of the simulation). The infusion rate is rapidly increased to about 1500 mL/h to maintain an SVV (%) of 15%. At *t* = 1 hr, the blood loss increases to 3mL/kg/min, and the controller increases the infusion rate to about 2250 mL/h to maintain the target SVV (%) of 15%.

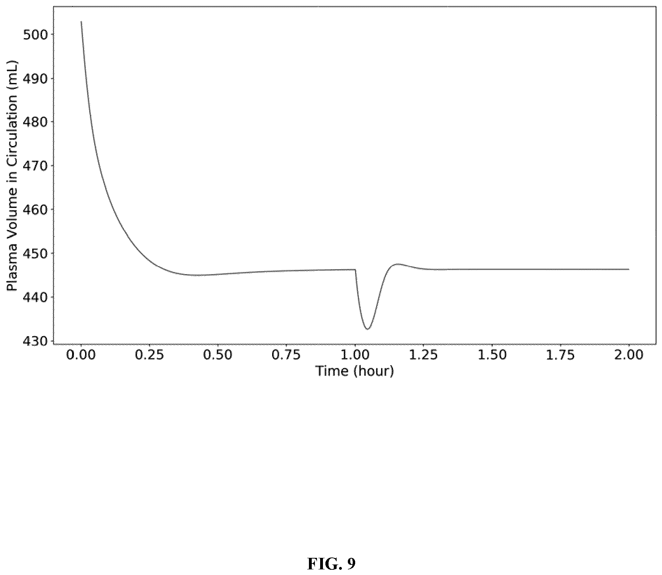

**FIG. 9** shows plasma volume in circulation versus time. The plasma volume in circulation rapidly decreases due to loss of blood in spite of fluid resuscitation until reaching an equilibrium value of about 450 mL. At *t* = 1 hr, the blood loss increases to 3mL/kg/min, and the controller increases the infusion rate to drive stroke volume variation measurements to the target value of 15%. The plasma volume in circulation remains about the same even after an increase in blood loss.

**EXAMPLE 5:** Animal study for fluid resuscitation.

The adaptive control framework of the disclosure was used to provide automated and semi-automated (clinical decision support) fluid resuscitation to five dogs in different hemorrhaging/hypovolemic scenarios.

Five mature intact Beagle dogs, determined to be healthy based on a physical examination and hemogram, were included in the experiment. The dogs were individually housed and provided commercial dry dog food and water ad libitum. Each dog and experiment was identified (Table 1). For example, S1-2 represents the second experiment performed on Subject S1. Studies on subjects were performed on different days. Individual trials on the same subject were performed on the same day and a stabilization period was used between trials. All dogs were euthanized with sodium pentobarbital (100 mg/kg, IV) upon completion of the experiments.

**TABLE 1. Study subject information and experiment summary. CH=controlled hypovolemia, UH=uncontrolled hypovolemia, RH=relative hypovolemia, RAH=relative and absolute hypovolemia**

| **Subject** | **Weight (kg)** | **Study 1** | **Study 2** |
|---|---|---|---|
| **S1** | 11.6 | CH | CH |
| **S2** | 7.1 | RAH | RAH |
| **S3** | 10.8 | UH | - |
| **S4** | 12.2 | RH | UH |
| **S5** | 9.3 | RH | UH |

**Animal Preparation:** Food but not water was withheld for 6 hours before each experiment. An intravenous catheter was transcutaneously positioned in the cephalic vein for administration of hydromorphone, 0.15 mg/kg IV. Anesthesia was produced ten minutes later by administering 3.5 to 6mg/kg IV propofol to facilitate orotracheal intubation, and initially maintained at a vaporizer setting of 1.5-2% isoflurane in oxygen. The dogs were positioned on their right side and mechanically ventilated at 10-12 breaths/min and 10-14 ml/kg tidal volume in order to maintain the end-tidal partial pressure of carbon dioxide (ET_{CO2}) between 38 and 48mmHg. To avoid potential changes in SVV, tidal volume settings for each subject was not changed during the study. Esophageal temperature was maintained (37°C) with temperature-controlled warm air blankets.

Vascular catheters were surgically placed in the left jugular vein and right carotid and femoral arteries after perivascular administration of 0.5-1.0 ml 2% lidocaine. The carotid or femoral artery catheter was connected to a FloTrac sensor with low-compliance fluid filled tubing. The FloTrac sensor was connected to a Vigileo monitor for determination and continuous monitoring of SVV. The FloTrac sensor was positioned and zeroed at the level of the sternum. The pressure line of the FloTrac sensor was flushed with 4 ml/hr of lactated ringer's solution (LRS). Heart rate was determined from a Lead II electrocardiogram (ECG). Criteria for obtaining accurate SVV recordings during mechanical ventilation were employed.

A 5 Fr Swan-Ganz catheter was percutaneously advanced via the right jugular vein (2 dogs) into the pulmonary artery under fluoroscopic guidance for measurement of cardiac output by thermodilution. Alternatively, cardiac output was determined by a previously implanted flow probe (3 dogs) positioned around the ascending aorta, proximal to brachio-cephalic trunk for continuous recording of cardiac output.

**Experimental Procedures:** Five dogs were subjected to 9 experiments. Lactated Ringer's solution (LRS) was administered as fluid resuscitation. A variety of experimental hypovolemic conditions were created in order to mimic various clinical conditions (Table 1). Absolute controlled hypovolemia (2 trials) was produced during 1.5 minimum alveolar concentration (MAC: 1.27% used throughout) of isoflurane anesthesia by withdrawing 15 ml/kg/15 minutes from either the right carotid or right femoral artery (S1-1). There was a 30-minute stabilization period between the end of the closed-loop fluid resuscitation (S1-1) and the beginning of the second hemorrhage, 40 ml/kg/30 minutes (S1-2). Closed-loop fluid administration was initiated within 10 minutes of completion of each blood withdrawal and continued until SVV reached a predetermined target range equal to or less than 13 ± 3%.

Absolute uncontrolled hypovolemia (S3-1, S4-2, S5-2; 3 trials), designed to simulate blood loss from a severed artery was produced by withdrawal of approximately 50% (40 ml/kg) of the dogs estimated blood volume (80 ml/kg) from the right carotid or right femoral artery over one hour. Five successive 8.0 ml/kg increments of blood were withdrawn continuously in increments that were completed at approximately 7-8, 18-20, 30-32, 43-45, and 60 minutes after initiating hemorrhage. Closed-loop fluid resuscitation was initiated 10 minutes after initiation of absolute uncontrolled hypovolemia (i.e., beginning of Stage 2 of blood withdrawal) and continued until SVV reached a predetermined target range equal to or less than 13 ± 3%.

Relative hypovolemia (2 trials) was produced by either increasing the inspired concentration of isoflurane to 2.0-2.5 MAC (S5-1, 1 trial) or administering sodium nitroprusside (5-10 mcg/kg/min; S4-1, 1 trial) until mean arterial blood pressure was ≤ 50 mm Hg. The target range SVV was set at 13 ± 3% for S4-1 and 5 ± 3% for S5-1. Relative and controlled absolute hypovolemia were produced by increasing the concentration of isoflurane (0.25-0.5%, 1.5-2.0 MAC multiples) in order to decrease MAP by ≥30% (S2-1, 1 trial) or administering sodium nitroprusside (1-15 mcg/kg/min; S2-2, 1 trial) followed by withdrawal of 15 ml/kg/minutes of blood. The target range SVV was set at 13 ± 3% for S2-1 and S2-2. The subject was resuscitated to the target SVV value in S2-1 and stabilized before initiating the second study (S2-2). Fluid resuscitation started 15 minutes after achieving relative and controlled absolute hypovolemia.

The closed-loop fluid resuscitation system was employed in a "partial automation" mode (clinical decision support) in two experimental trials, one involving absolute uncontrolled hemorrhage (S4-2) and one involving relative hypovolemia from sodium nitroprusside administration (S4-1), where the system displayed the recommended infusion rate every minute and the user manually changed the infusion rate. A Horizon NXT Modular Infusion System pump was used in the partial automated mode. While the system was able to provide infusion rate recommendations more frequently, an update interval of 1 minute was chosen to allow sufficient time for the clinical staff to adjust the pump settings manually. Measured SVV values were filtered in all studies, including automated and partially automated modes, using a 1-minute moving window averaging to remove noise. The adaptive controller was implemented using a neural network with sigmoidal basis functions to use as the function approximator.

The subject continuously received a fluid infusion and the control system changed the infusion rate every few seconds in response to changes in SVV. Vigileo transmitted the most recent value of the SVV measurement every 2 seconds using serial communication. The adaptive control framework was implemented on a laptop. The laptop was connected to the Vigileo using a Serial to USB cable. The closed-loop fluid resuscitation system used a 1-minute moving window averaging to remove noise. The control algorithm was implemented in the Python programming language running on a laptop with the Linux operating system. Measurements from Vigileo were recorded by the laptop using serial communication. The closed-loop fluid resuscitation algorithm computed an infusion rate every 11 seconds using the average SVV values in the past 1-minute. The infusion rate was sent by the laptop to an infusion pump (supporting flow rates from 0.06 to 4200 ml/hr) using a USB connection.

**Performance Metrics:** Performance metrics that are of clinical relevance were defined in order to assess the performance of the closed-loop fluid resuscitation system. Specifically, T_{target} was defined as the duration from start of fluid administration to restoration of an acceptable SVV target range.

We defined the acceptable SVV target range to be equal to 13 ± 3% with the exception of two experiments, namely, S5-2 (uncontrolled hypovolemia) and S5-1 (relative hypovolemia), where the acceptable SVV target range was 10 ± 3% and 5± 3%, respectively. The R_{in range} was defined as the percentage of time that SVV target value stayed in the acceptable range (i.e., duration of time SVV stayed in the acceptable range divided by total duration of resuscitation). Other performance metrics included minimum and maximum infusion rates denoted by uₘᵢₙ and uₘₐₓ, respectively, total infused volume to reach the acceptable SVV target range denoted by V_{target}, and, in absolute hypovolemia experiments, the ratio of total fluid volume infused to total blood loss denoted by Vᵣₐₜᵢₒ. To ensure that the subject can be maintained at the acceptable SVV target range, the resuscitation continued after reaching the acceptable SVV target range, and hence, V_{target} and total infused volume are not equal. Continued resuscitation after reaching V_{target} lasted approximately 15 minutes on average. The stabilization period between different trials on the same subject started after the completion of the fluid resuscitation of the previous trial.

**Controlled Hypovolemia:** Absolute hypovolemia during 1.5 MAC isoflurane anesthesia decreased MAP from 100 to 86 mmHg (dog S-1) and from 109 to 54 mm Hg (S1-2) after withdrawal of 15 ml/kg and then 40 ml/kg of blood respectively (Table 2). Heart rate increased and cardiac output decreased after withdrawal of 15 ml/kg and 40 ml/kg of blood (Table 2). In addition, the SVV increased after withdrawal of 15 ml/kg and 40 ml/kg of blood. The SVV returned to the target range (13% ± 3) after the administration of 7 ml/kg and 66 ml/kg of LRS, respectively (Table 2). The total infused volumes were 189 ml (S1-1: Vᵣₐₜᵢₒ = 1.1) and 925 ml (S1-2: Vᵣₐₜᵢₒ = 2), respectively.

**Table 2. Hemodynamic data and performance metrics for controlled hypovolemia study. T_{target} denotes the duration from start of fluid administration to restoration of an acceptable SVV target range, uₘᵢₙ and uₘₐₓ denote minimum and maximum infusion rates, respectively, and V_{target} denotes total infused volume to reach the acceptable SVV target range.**

| | **Baseline** | | **Before Resuscitation** | | **After Resuscitation** | |
|---|---|---|---|---|---|---|
| **ml/kg bled** | 15 | 40 | 15 | 40 | 15 | 40 |
| **CO (L/min)** | 1.5 | 2.1 | 1.3 | 0.8 | 2.1 | 1.6 |
| **MAP (mmHg)** | 100 | 109 | 86 | 54 | 109 | 63 |
| **HR (bpm)** | 85 | 108 | 107 | 187 | 108 | 154 |
| **SVV (%)** | 6 | 15 | 19 | 42 | 13 | 16 |

### Performance Metrics

| **Study** | **T_{target} (min)** | **uₘᵢₙ (ml/kg/hr)** | **uₘₐₓ (ml/kg/hr)** | **V_{target} (ml/kg)** |
|---|---|---|---|---|
| **S1-1** (15ml/kg) | 6.6 | 26 | 64 | 7 |
| **S1-2** (40 ml/kg) | 50.3 | 70 | 87 | 66 |

**Uncontrolled Hypovolemia:** Closed-loop fluid administration was initiated during the second stage of blood withdrawal, and continued throughout hemorrhage (S3-1, S4-2, S5-2). Closed-loop fluid administration was stopped approximately 30 minutes after the end of the last (fifth) stage of hemorrhage as this exceeds the average time for fluid equilibration with the interstitial fluid compartment. Mean arterial blood pressure and cardiac output decreased and heart rate increased during the initial 3-4 stages of uncontrolled hypovolemia (Table 3). Heart rate increased throughout hemorrhage and remained elevated throughout hemorrhage and fluid administration (Table 3). The SVV increased during the first stage of uncontrolled hypovolemia and returned toward baseline values thereafter (Table 3). Total infused volumes for S3, S4, and S5 were 1,092 ml (Vᵣₐₜᵢₒ = 2.5), 1,243 ml (Vᵣₐₜᵢₒ = 2.8), and 548 ml (Vᵣₐₜᵢₒ = 1.4), respectively. The fluid administration system was used in a partially-automated (human-in-the-loop) mode for S4, where the recommended infusion rate was displayed to the user every minute and the user manually changed the infusion rate to the recommended value.

**Table 3. Hemodynamic data and performance metrics for uncontrolled hypovolemia study. uₘᵢₙ and uₘₐₓ denote minimum and maximum infusion rates, respectively, and R_{in range} the percentage of time that SVV target value stayed in the acceptable range.**

| | **Baseline** | **Stage 1** | **Stage 2** | **Stage 3** | **Stage 4** | **Stage 5** | **End** |
|---|---|---|---|---|---|---|---|
| **CO (L/min)** | 1.5 (1.1-1.7) | 0.8 (0.5-1.5) | 0.8 (0.3-1.4) | 1.0 (0.4-1.4) | 0.9 (0.4-1.7) | 1.0 (0.7-1.7) | 1.3 (1.1-2.0) |
| **MAP (mmHg)** | 81 (62-85) | 59 (55-91) | 66 (35-87) | 65 (45-67) | 68 (50-72) | 68 (53-78) | 59 (44-83) |
| **HR (bpm)** | 118 (113-121) | 134 (120-171) | 132 (108-170) | 132 (117-174) | 135 (117-170) | 139 (116-168) | 137 (107-167) |
| **SVV (%)** | 8 (6-12) | 21 (17-25) | 17 (12-19) | 15 (11-16) | 14 (11-16) | 15 (10-15) | 10 (8-18) |

### Performance Metrics

| **Dog** | **uₘᵢₙ (ml/kg/hr)** | **uₘₐₓ (ml/kg/hr)** | **R_{in range} (%)** |
|---|---|---|---|
| **S3-1** | 44 | 115 | 54 |
| **S4-2** | 40 | 82 | 71 |
| **S5-2** | 39 | 64 | 100 |

**Relative Hypovolemia from Vasodilatation:** Dogs were made hypotensive by increasing the inspired concentration of isoflurane (S5-1 dog) or by administering sodium nitroprusside (S4-1) until mean arterial blood pressure was less than or equal to 50 mmHg. The target range SVV was set at 13 ± 3% for S4-1 and 5 ± 3% for S5-1. The administration of isoflurane or sodium nitroprusside decreased mean arterial blood pressure and increased heart rate and either did not change or increased cardiac output, respectively (Table 4). SVV increased in both dogs (Table 4). Closed-loop fluid resuscitation increased cardiac output and decreased SVV in both dogs. Arterial blood pressure increased during fluid administration in the dog administered sodium nitroprusside (S4-1) but not in the dog administered isoflurane (S5-1) (Table 4). Total infused volumes were 187 ml (nitroprusside: 15 ml/kg) and 265 ml (isoflurane: 28 ml/kg).

**Table 4. Hemodynamic data and performance metrics for the relative hypovolemia study. T_{target} denotes the duration from start of fluid administration to restoration of an acceptable SVV target range, uₘᵢₙ and uₘₐₓ denote minimum and maximum infusion rates, respectively, V_{target} denotes total infused volume to reach the acceptable SVV target range, and R_{in range} the percentage of time that SVV target value stayed in the acceptable range. I = isoflurane; SN = sodium nitroprusside**

| | **Baseline** | **Before Resuscitation** | **After Resuscitation** |
|---|---|---|---|
| **CO (L/min)** | 0.8 I; 1.4 SN | 0.8 I; 1.6 SN | 1.4 I; 2.0 SN |
| **MAP (mmHg)** | 62 I; 69 SN | 45 I; 47 SN | 35 I; 94 SN |
| **HR (bpm)** | 84 I; 89 SN | 87 I; 115 SN | 89 I; 126 SN |
| **SVV (%)** | 7 I; 9 SN | 10 I; 17 SN) | 6 I; 8 SN |

### Performance Metrics

| **Study** | **T_{target} (min)** | **uₘᵢₙ (ml/kg/hr)** | **uₘₐₓ (ml/kg/hr)** | **V_{target} (ml/kg)** | **R_{in range} (%)** |
|---|---|---|---|---|---|
| **S4-1** | 3 | 11 | 67 | 3 | 100 |
| **S5-1** | 4 | 63 | 73 | 5 | 90 |

**Relative Hypovolemia and Controlled Hypovolemia:** Increasing the isoflurane concentration from 1.5 to 2.5 MAC (S2-1) or administration of sodium nitroprusside (S2-2) was followed by blood withdrawal (15 ml/kg/15 minutes). The sodium nitroprusside infusion rate started at 1 mcg/kg/min and was increased to 15 mcg/kg/min. The target range SVV was set at 13 ± 3%. Absolute hypovolemia (15 ml/kg/15 min) during 2.5 MAC isoflurane anesthesia decreased MAP from 60 mmHg before blood withdrawal to 39 mm Hg after blood withdrawal (S2-1). In addition, MAP increased from 39 mmHg to 46 mmHg after a 15-minute stabilization period. Heart rate changed minimally (110 vs. 112 bpm) and cardiac output decreased 20% (1.0 vs. 0.8 L/min) after the production of relative hypovolemia combined with controlled hypovolemia (15 ml/kg/15 min). The SVV increased from 13% at 1.5 MAC to 21% at 2.5 MAC and then to 41% after withdrawal of 15 ml/kg of blood. The SVV decreased to 26% after a 15-minute stabilization period. The SVV returned to the target range (13% ± 3) at 43 minutes (T_{target}) after the administration of 78 ml/kg of LRS. Total infused volume was 573 ml (Vᵣₐₜᵢₒ = 5.4). The maximum fluid administration rate (uₘₐₓ) was 113 ml/kg/hr. Heart rate decreased (112 to 99) and cardiac output increased to above the baseline value (1.0 vs. 1.2) after LRS administration but MAP remained relatively unchanged (43 mm Hg) until the isoflurane concentration was decreased.

Absolute hypovolemia (15 ml/kg/15 min) during 1.5 MAC and sodium nitroprusside administration decreased MAP from 113 to 92 mmHg prior to blood withdrawal (S2-2). MAP was 101 mmHg after blood withdrawal. Heart rate changed minimally and cardiac output decreased 25% (1.3 vs. 1.0 L/min) after the production of relative hypovolemia combined with controlled hypovolemia (15 ml/kg/15 min). The SVV increased from 10% to 15% after sodium nitroprusside administration and then to 22% after withdrawal of 15 ml/kg of blood. The SVV returned to the target range (13% ± 3) at 26 minutes (T_{target}) after the administration of 46 ml/kg of LRS. The maximum fluid administration rate (uₘₐₓ) was 108 ml/kg/hr. Cardiac output increased to above the baseline value (1.3 vs. 2.1) after LRS administration and MAP increased to near the baseline value (Table 5). Total infused volume was 400 ml (Vᵣₐₜᵢₒ = 3.7).

**Table 5. Hemodynamic data and performance metrics for the relative hypovolemia and controlled hypovolemia study. T_{target} denotes the duration from start of fluid administration to restoration of an acceptable SVV target range, uₘᵢₙ and uₘₐₓ denote minimum and maximum infusion rates, respectively, V_{target} denotes total infused volume to reach the acceptable SVV target range, and R_{in range} the percentage of time that SVV target value stayed in the acceptable range. I = isoflurane; SN = sodium nitroprusside**

| | **Baseline** | **Before Resuscitation** | **After Resuscitation** |
|---|---|---|---|
| **CO (L/min)** | 1.0 I; 1.3 SN | 0.8 I; 1.0 SN | 1.2 I; 2.1 SN |
| **MAP (mmHg)** | 93 I; 113 SN | 46 I; 101 SN | 40 I; 115 SN |
| **HR (bpm)** | 110 I; 134 SN | 112 I; 114 SN | 99 I; 108 SN |
| **SVV (%)** | 13 I; 10 SN | 26 I; 22 SN | 15 I; 21 SN |

### Performance Metrics

| **Study** | **T_{target} (min)** | **uₘᵢₙ (ml/kg/hr)** | **uₘₐₓ (ml/kg/hr)** | **V_{target} (ml/kg)** | **R_{in range} (%)** |
|---|---|---|---|---|---|
| **S2-1** | 43 | 88 | 113 | 78 | 100 |
| **S2-2** | 26 | 66 | 108 | 46 | 53 |

The closed-loop fluid resuscitation system used the compartmental modeling framework to compute fluid infusion rates. The two-compartment model of the microvascular exchange system involved parameters that are generally unknown and different from patient to patient. In addition, the two-compartment model was only an approximation of the fluid distribution in the body, resulting in modeling error.

The adaptive control fluid resuscitation algorithm uses the compartmental characteristics of the fluid distribution in the body. To address modeling error and unknown parameters of the compartmental model governing fluid distribution, the adaptive algorithm used a "function approximator." The function approximator was characterized by a set of parameters, which were continuously estimated by the closed-loop system in real-time. The closed-loop fluid resuscitation system re-computes the fluid infusion rate periodically. The function approximator used the values of infusion rates and SVV measurements to estimate the dynamics of fluid distribution. The controller performance was evaluated with computer simulations on a two-compartment model prior to conducting the animal study. The results presented are the first attempt to use the disclosure in live subjects.

Data from this study confirmed that an adaptive closed-loop fluid administration system based on a compartmental model of fluid distribution provided targeted goal-directed fluid therapy in dogs subjected to experimental conditions that mimicked clinical scenarios of absolute (controlled, uncontrolled), relative hypovolemia or a combination of relative hypovolemia and absolute controlled hypovolemia. Stroke volume variation was restored and maintained to within a preselected normal target range in less than one hour after initiating fluid administration. Larger volumes of blood loss (40 vs.15 ml/kg) increased the Vᵣₐₜᵢₒ required to restore SVV to the target range but remained below amounts based upon lactated ringer's solution (LRS) distribution.

The adaptive control algorithm was based on physiology, and the parameters of the model were estimated in real-time. The framework provided a mechanism to account for inter-patient and intra-patient variability in the fluid resuscitation process.

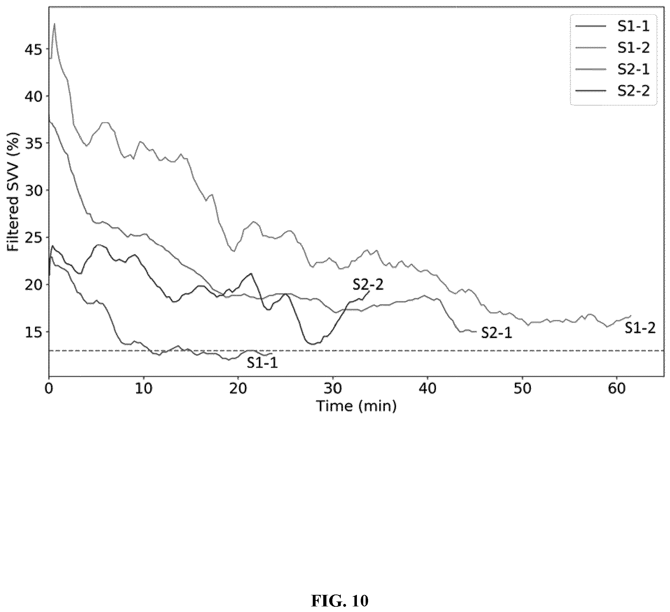

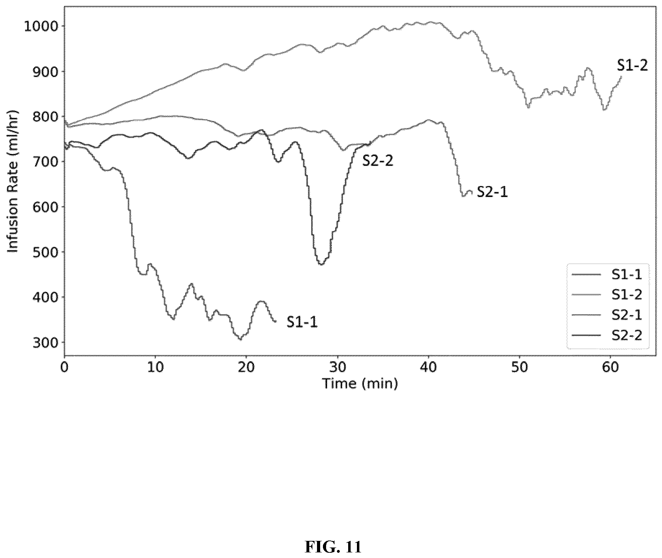

**FIGs. 10** **AND 11** show the results for 2 canine subjects S1 and S2 (total of 4 studies) experiencing controlled hemorrhage. **FIG. 10** shows changes in filtered SVV (%) versus time, and **FIG. 11** shows changes in infusion rate (mL/hr) versus time. The target SVV was 13%. In study S1-1, the infusion rate dropped from 750 mL/hr to about 400 mL/hr to maintain an SVV of 13%. Once reaching the target SVV (%), the infusion rate fluctuated between 300 - 400 mL/hr to maintain an SVV of 13% In study S1-2, the infusion rate started from about 800 mL/hr increased to about 1000 ml/hr and decreased to about 850 ml/hr. In study S2-1, the infusion rate started from about 800 mL/hr and in the end reached about 650 ml/hr. In study S2-2, the infusion rate started from about 750 mL/hr and in about 30 minutes reached about 500 ml/hr but increased to 750 ml/hr.

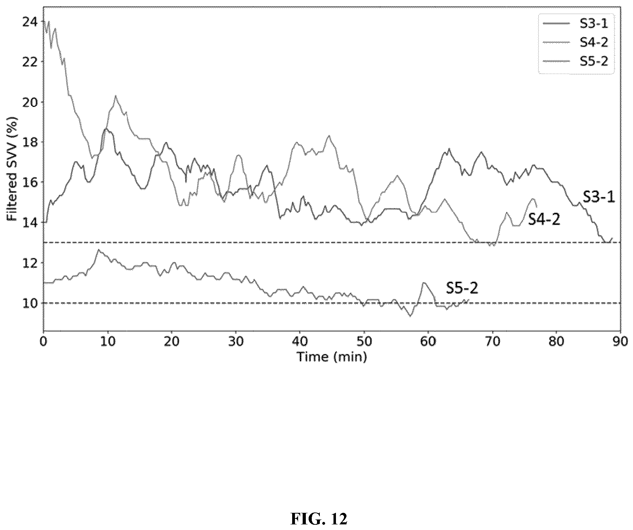

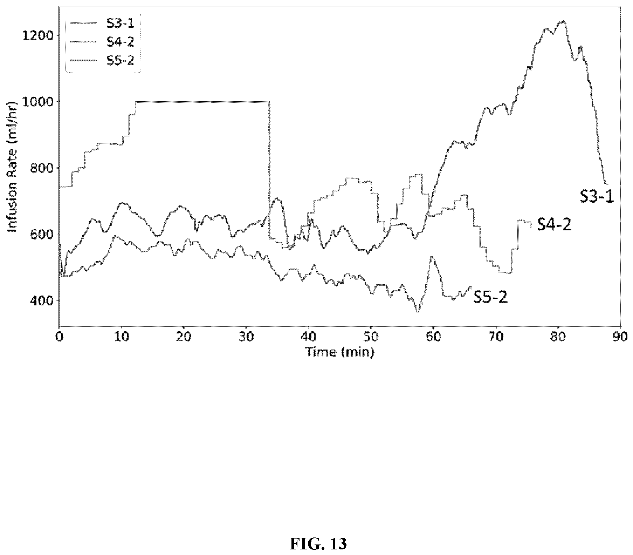

**FIGs. 12** **AND 13** shows the results for 3 canine subjects S3, S4, and S5 experiencing uncontrolled hemorrhage. **FIG. 12** shows changes in filtered SVV (%) versus time, and **FIG. 13** shows changes in infusion rate versus time. The target SVV was 13% in studies S3-1 and S4-2 and was 10% in study S5-2. In study S4-2, the partial automation (clinical decision support) was used where every 1 minute the clinician used fluid rates recommended by the system to manually change the infusion rate on the pump. In the other two studies S3-1 and S5-2 the fully automated closed-loop system was used. In study S3-1, the infusion rate started from about 600 mL/hr and increased to about1200 mL/hr to drive SVV to 13% and then dropped to 750 mL/hr. In study S4-2, the infusion rate started from about 750 mL/hr increased to 999 mL/hr and then decreased to about 600 mL/hr to drive SVV to 13%. In study S5-2, the infusion rate started from about 500 mL/hr and fluctuated between 400-600 mL/hr to drive SVV to the target value of 10%.

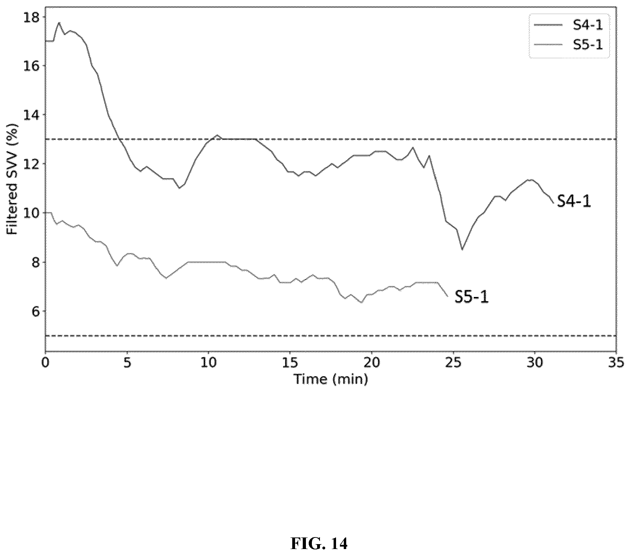

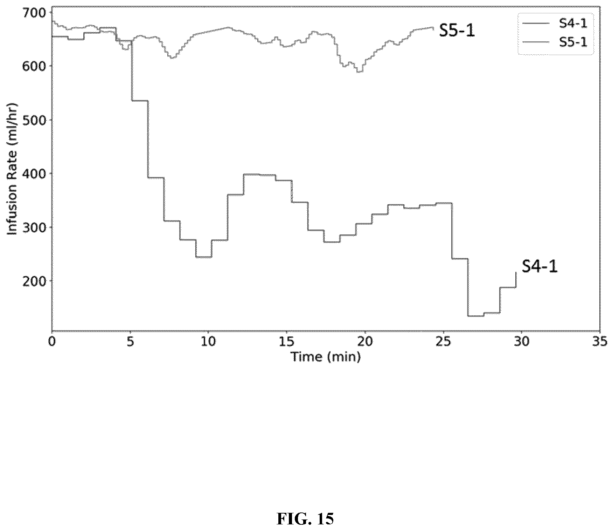

**FIGs. 14** **and** **15** shows the fluid resuscitation of 2 canine subjects S4 and S5 that were hypotensive as a result of administration of sodium nitroprusside (in S4-1) and increase in the inhalant anesthetic isoflurane (in S5-1). In study S5-1, the fully automated closed-loop system was used while in study S4-1 the partially automated (clinical decision support) system was used where the recommended infusion rate was displayed every 1 minute to the user and the user changed the infusion rate on the pump manually. **FIG. 14** shows changes in filtered SVV (%) versus time, and **FIG. 15** shows changes in infusion rate versus time. The target SVV was 13% in S4-1 and 5% in S5-1. In S4-1, the infusion rate was started at about 650 mL/hr and decreased to between 150-350 mL/hr once the target 13% was reached. In S5-1, the infusion rate started at about 700 mL/hr and was kept close to this value until 25 minutes into the study, when the study was terminated as SVV approached the desired SVV of 5%.

### EXAMPLE 6: Higher-level controller.

The higher-level controller was designed as a rule-based expert system and served to:
i) monitor the lower-level controller (fluid resuscitation module and/or cardiovascular drug administration module) function for possible anomalies;
*ii)* monitor the patient's general status and response to fluid therapy and/or cardiovascular drug administration;
*iii)* in the case of closed-loop mode decide to engage fluid resuscitation module, cardiovascular drug administration module, or both, and the timing of engagement of the modules;
iv) handle scenarios related to sensor failure or infusion rate exceeding maximum safe limit;
v) modify the lower-level controller (fluid resuscitation module or cardiovascular drug administration module) states in the case of clinical decision support if the user disagrees with the computed infusion rate;
vi) provide clinical decision support to address potential problems; and
*vii)* in clinical decision support mode, notifying the user with the new infusion rate when the infusion rate needs to be updated.

**Maintaining Infusion Rate in Safe Range.** If the computed infusion rate by the lower-level controller (fluid resuscitation module and/or cardiovascular drug administration module) was larger than the maximum safe rate specified by the user, the higher-level controller reset the lower-level controller associated with unallowable infusion rate, that is, it reinitialized the internal states of the lower-level controller including function approximator weights (or coefficients) to the default values (i.e., changed *w*ₚ, *w*_{q}, and *x*_{c} to their values at *t=0).*

**Warning the User.** If the total volume delivered to the patient exceeded a set threshold (e.g., 2000 mL or 10,000 mcg), the higher-level controller warned the user of the risk of complications.

**Monitoring the Lower-Level Controller.** If performance degradation in the closed-loop system was detected, the higher-level controller notified the user through an audio-visual alarm, and the higher-level controller stopped the lower-level controller. Performance degradation was defined as: i) a rapid change in weights (or coefficients) of the function approximator, that is, $\frac{d \left|W\right|}{dt} > threshold$ (e.g., 0.1, or 1, 10 etc.); or *ii)* the number of resets of the lower-level controller by the higher-level controller exceeding a threshold value (e.g., 1, 2, 3, 4, etc.), or *iii)* the absolute difference between the measured value (e.g., SVV or mean arterial pressure) and the target value did not decrease in a period of time set by user while the infusion was continuously increased. In case of sensor failure or unavailability of measurements, the higher-level controller disabled the lower-level controller and set the infusion rate to a backup the infusion rate set by the user.

**Deciding When to Engage Fluid Resuscitation and Cardiovascular Drug Administration Modules.** In certain scenarios such as sepsis, it is preferable to provide fluid resuscitation first, and if unsuccessful in achieving an acceptable condition, administer a cardiovascular drug such as a vasopressor. The higher-level controller first engaged the fluid resuscitation module and monitored the patient. After a period of time when a clinically relevant hemodynamic variable (e.g., mean arterial pressure) was not improved in spite of fluid resuscitation (e.g., the mean arterial pressure was not in the acceptable range of 65-80 mmHg after 30 minutes), the higher-level controller engaged the cardiovascular drug administration module to administer a vasopressor. In some embodiments, the clinician instructed the higher-level controller to engage the cardiovascular drug administration module while the fluid resuscitation module was already running. In some embodiments, the clinician instructed the higher-level controller to engage the fluid resuscitation module while the cardiovascular administration module was already running. In this scenario, stroke volume variation was not improved by only administering a cardiovascular drug (e.g., a vasopressor) and the higher-level controller engaged the fluid resuscitation module.

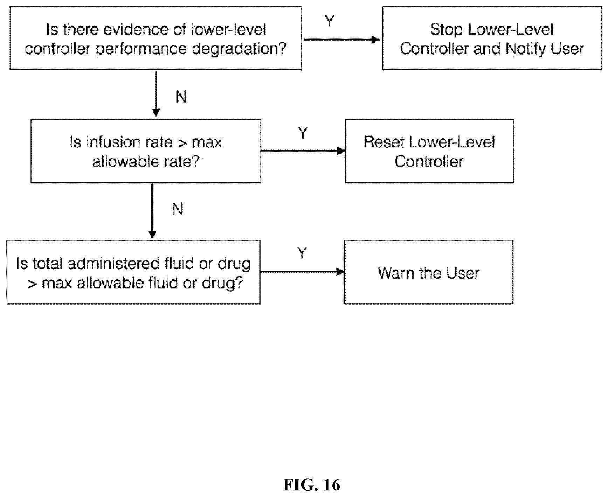

**FIG. 16** illustrates the components of the higher-level controller for the closed-loop fluid resuscitation and/or cardiovascular drug administration system. The higher-level controller can monitor the fluid resuscitation module and/or the cardiovascular drug administration depending on whether the system is used for fluid resuscitation only, for cardiovascular drug administration only, or combined fluid resuscitation and cardiovascular drug administration.

In the clinical decision support system case, the lower-level controller (fluid resuscitation module or cardiovascular drug administration module or both) sent newly computed infusion rate to the higher-level controller. Specifically, we considered 3 scenarios: i) fluid resuscitation module only; ii) cardiovascular drug administration module only; and iii) fluid resuscitation module and cardiovascular drug administration module working concurrently. In each scenario, the lower-level controller sent newly computed infusion rate to the higher-level controller. The higher-level controller compared the new rate with the last user approved rate (i.e., for a fluid resuscitation, the newly computed fluid infusion rate was compared with the last user-approved fluid infusion rate, and for cardiovascular drug administration, drug infusion rate was compared with the last user-approved drug infusion rate).

If the difference between the new infusion rate and the last user-approved rate was less than the threshold value (e.g., 25% of the last approved rate), then the infusion rate was not changed, the user was not notified, and the lower-level controller continued its operation. If the difference between the new infusion rate and the last user-approved rate was higher than some threshold (e.g., 25% of the last approved rate), then the recommended infusion rate was displayed to the user through the graphical user interface. If the user accepted the infusion rate, the higher-level controller allowed the lower-level controller to continue its operation and the infusion rate was updated to the new rate. However, if the user changed the recommended infusion rate to a different value, the higher-level controller reset the controller and set the weights of the function approximator such that the infusion rate computed by the lower-level controller matched the infusion rate entered by the user. The infusion rate was also updated to the rate provided by the user. Given the user specified infusion rate, the weights of the function approximator was chosen such that ${w}_{q , new} \left(0\right) = {w}_{q} \left(0\right)$ ${w}_{p , new}^{T} \left(0\right) P \left(z\left(0\right)\right) = \left(new infusion\right) \left(1+{w}_{q}^{T}\left(0\right)Q\left(z\left(0\right)\right)\right)$

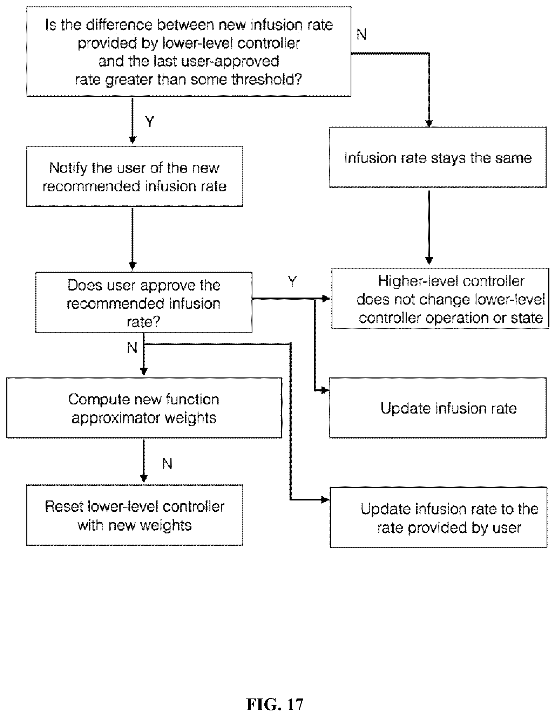

**FIG. 17** illustrates the components of the higher-level controller for the clinical decision support case.

### EXAMPLE 7: Computer simulations for vasopressor administration.

Adaptive control frameworks were used to simulate cardiovascular drug (vasopressor) administration of a 70 kg patient experiencing sepsis and the associated hypotension. The goal was to maintain the mean arterial pressure at 65. A Δ*t* of 0.1 minute (6 seconds) was used for the simulations and *β*₁ = 6e-5, *β*₂ = 13e-6, and *n*_{node} = 8. Only the vasopressor was administered to maintain a mean arterial pressure of 75 mmHg. The patient model involved a cardiovascular model to model hemodynamics and a compartmental model to model fluid distribution.

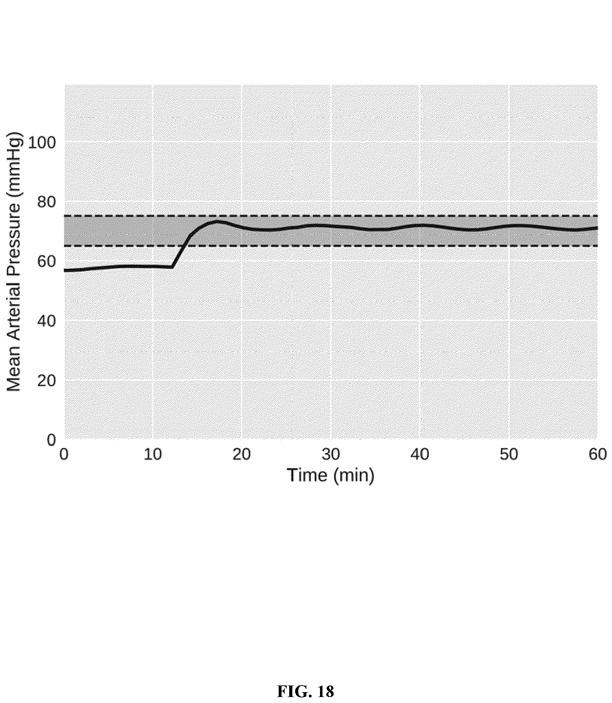

**FIG. 18** **shows** mean arterial pressure (MAP) versus time. The target MAP was 75 mmHg, and the 65-75 mmHg region is highlighted on the graph. MAP started at below 60 mmHg, and changes with the introduction of vasopressor epinephrine. The MAP increased to the target value of 75mmHg.

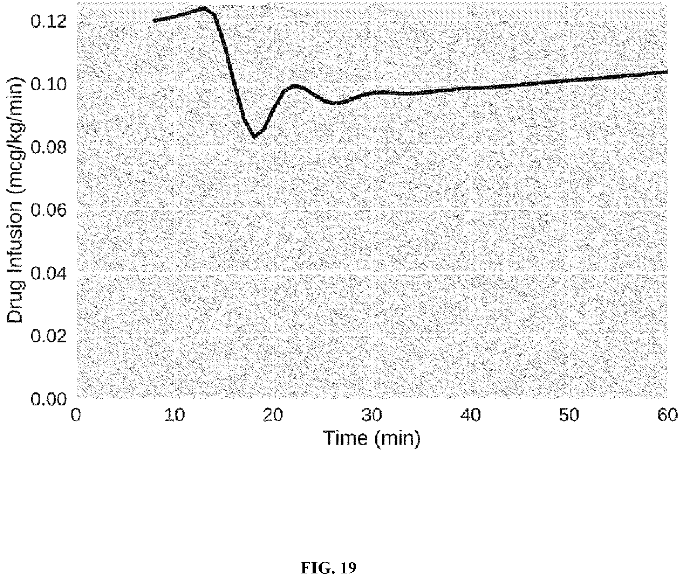

**FIG. 19** shows **infusion** rates computed by the adaptive control framework. The initial infusion rate was chosen by the user to be 0.12 mcg/kg/min at *t*=8 (start of the vasopressor administration). The infusion rate gradually increased and then started to decrease as MAP started to approach the target MAP of 75 mmHg reaching a low infusion rate of 0.85 mcg/kg/min. The infusion rate then started to gradually increase to maintain MAP of 75 mmHg.

### EXAMPLE 8: Computer simulations for fluid administration using mean arterial pressure.

Adaptive control frameworks were used to simulate fluid resuscitation of a 70 kg patient with hypotension as a result of sepsis. The goal was to maintain the mean arterial pressure at 75 mmHg. A Δ*t* of 0.1 minute (6 seconds) was used for the simulations and *β*₁ = 0.02, *β*₂ = 0.04, and *n*_{node} = 8. The patient model involved cardiovascular modeling to model hemodynamics and a compartmental model to model fluid distribution.

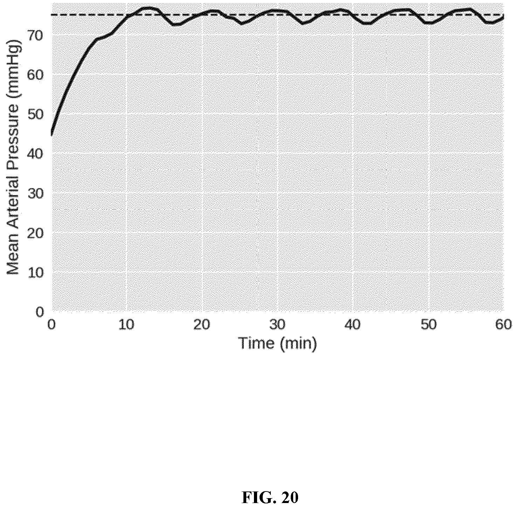

**FIG. 20** shows mean arterial pressure (MAP) versus time. The target MAP is 75 mmHg and the 65-75mmHg region is highlighted on the graph. MAP starts at approximately 45 mmHg, and changes with the introduction of crystalloid fluid. The MAP increases to the target value of 75 mmHg.

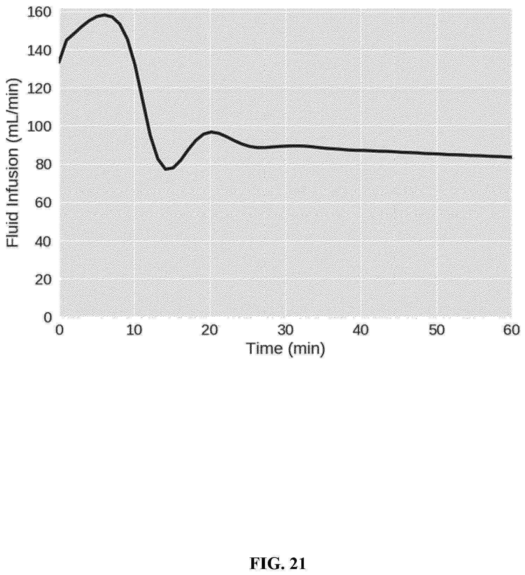

**FIG. 21** shows infusion rates computed by the adaptive control framework. The initial infusion rate was chosen was approximately 140 mL/min. The infusion rate gradually increased to 160 mL/min and then decreased to around 80 ml/min.

### EXAMPLE 9: Computer simulations for fluid administration and cardiovascular drug administration.

Adaptive control frameworks were used to simulate fluid resuscitation and cardiovascular drug (vasopressor epinephrine) administration for a 70 kg patient with hypotension as a result of sepsis. The goal was to maintain the mean arterial pressure at 75 mmHg and stroke volume variation (SVV) of 12%. A Δt of 0.1 min (6 seconds) was used for the simulations and *β*₁ = 0.02, *β*₂ = 0.04, and *n*_{node} = 8 for the fluid resuscitation module and *β*₁ = 6e-5, *β*₂ = 13e-6, and *n*_{node} = 8 for the cardiovascular drug administration module. The simulation involved crystalloid fluid and epinephrine (vasopressor). The fluid resuscitation module used SVV data to compute fluid infusion rates, and the cardiovascular drug administration module used mean arterial pressure (MAP) to compute the vasopressor infusion rates. The higher-level controller first engaged the fluid resuscitation module to provide fluid infusion. After 30 minutes, the higher-level controller engaged the cardiovascular administration module as mean arterial pressure was not improved after fluid infusion. The patient model involved cardiovascular modeling to model hemodynamics and compartmental model to model fluid distribution. The start of simulation is when the patient condition rapidly deteriorates.

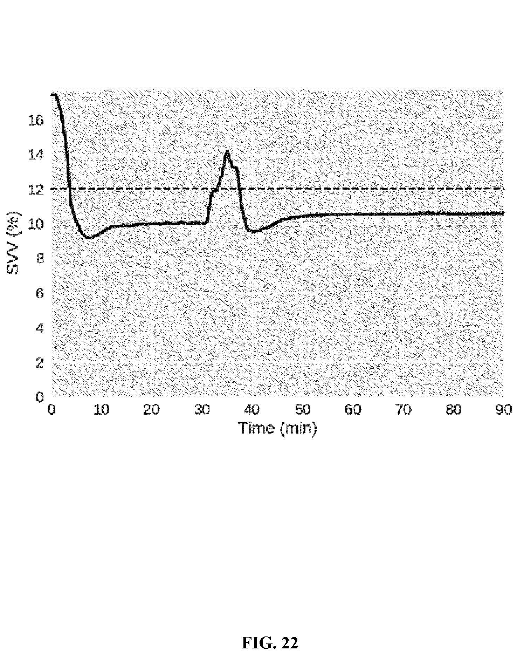

**FIG. 22** shows stroke volume variation (SVV (%)) versus time. The target stroke volume variation was 12%. The SVV (%) started at about 18 %, and was reduced to 10% after fluid resuscitation. SVV momentarily increased due to the administration of epinephrine (a transient vasodilatory effect). In the end of the simulation, SVV was approximately 11% and remained close to the target SVV value of 12%.

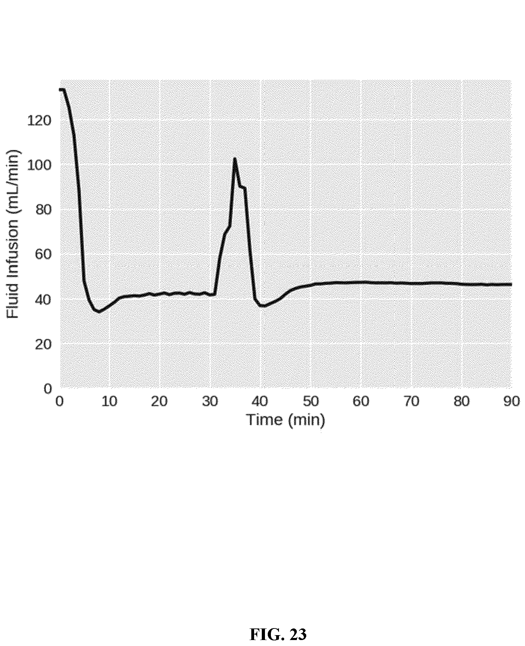

**FIG. 23** shows fluid infusion rates computed by the fluid resuscitation adaptive control framework. The infusion rate started at approximately 130 mL/min and was reduced to 40 mL/min after SVV was at an acceptable range. The sudden increase in SVV at t=30 min resulted in an increase in infusion rate which then reduced back to 40 mL/min after SVV was reduced.

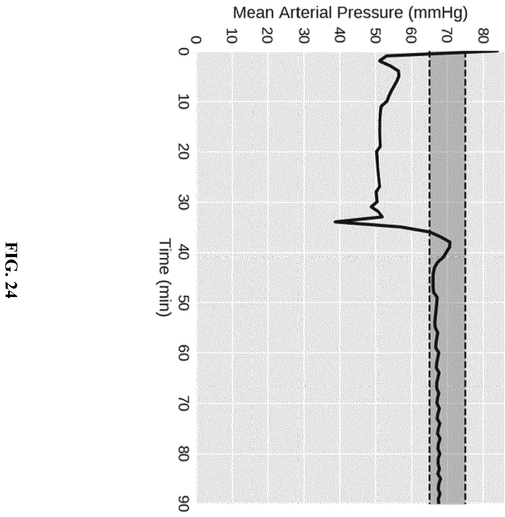

**FIG. 24** shows mean arterial pressure (MAP) versus time. The target MAP was 75 mmHg and the 65-75 mmHg region is highlighted on the graph. MAP dropped to 50 mmHg as the patient condition deteriorated. After 30 minutes, when the cardiovascular drug administration module was engaged by the higher-level controller, MAP started to increase and approached the set value of 75 mmHg. At the end of the simulation, MAP was approximately 70 mmHg and remained close to the target MAP value of 75 mmHg.

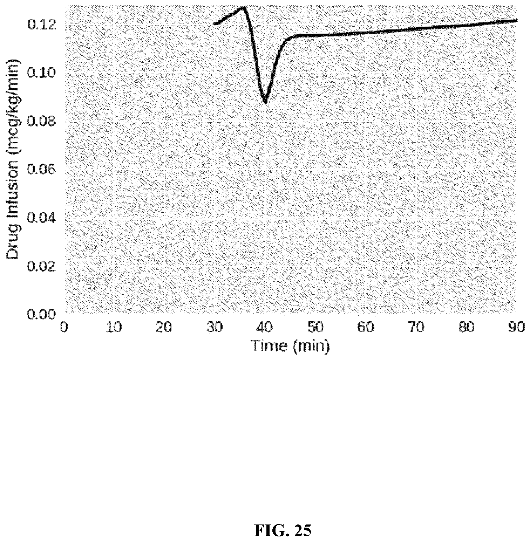

**FIG. 25** shows vasopressor infusion rates computed by the cardiovascular administration adaptive control framework. The initial infusion rate was chosen to be approximately 0.12 mcg/kg/min. After a slight increase, the rate decreased to approximately 0.9 mcg/kg/min and then stabilized around 0.12 mcg/kg/min as MAP is maintained close to 70 mmHg.

### EXAMPLE 10: Fluid resuscitation and cardiovascular drug administration system implemented on hardware.

A hardware platform was developed to implement the fluid resuscitation system and the cardiovascular drug administration system. The system is composed of a processing module and an associated touchscreen. The processing module is able to receive data from a hemodynamic monitor (invasive or non-invasive measurement) or a blood pressure module (either invasive measurement through a pressure transducer connected to an arterial line or non-invasive measurement) through a wired connection. In addition, the processing module is able to send real-time infusion rate data through a wired connection to an infusion pump. The touchscreen is used for displaying graphs such as received measurements over time and infusion rate over time. The main dashboard also displays current measurement, current infusion rate, and total volume of fluid/drug infused. The touchscreen allows the user to specify different parameters required by the system. For example, the user sets the target measurement value, initial infusion rate, backup infusion rate (in case of missing sensor data, the closed-loop system is disengaged and transitions to backup infusion rate until the user takes over) maximum infusion rate and other variables. In the case of clinical decision support, the touchscreen is used to communicate the new infusion rate value to request user's approval or give the user the ability to modify the rate. Once the user approves the infusion rate, the processing module updates the infusion rate of the infusion pump to the infusion rate approved by the user.

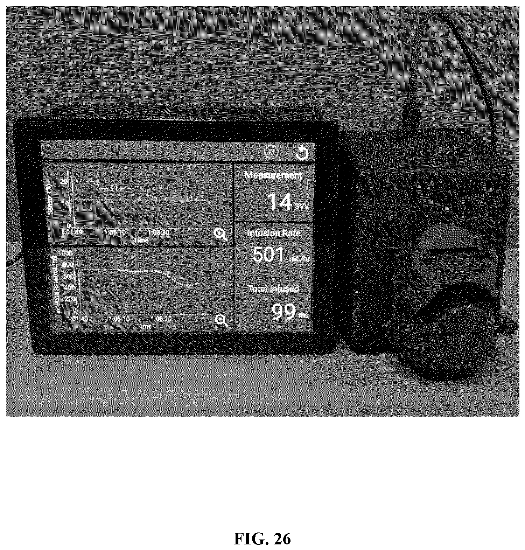

**FIG. 26** illustrates a picture of the hardware platform.

## Claims

1. A system for fluid resuscitation and/or cardiovascular drug administration, wherein the system:
(i) comprises at least one medical monitoring device, at least one infusion pump and
**characterized in that**
it comprises a hierarchical control architecture system, wherein the hierarchical control architecture system comprises at least one adaptive controller as the lower level controller and a logic-based controller as the higher level controller; and
(ii) is configured for performing a method comprising:
a) initiating an infusion of a fluid and/or a cardiovascular drug into a subject at an infusion rate;
b) receiving hemodynamic sensor data of the subject from the at least one medical monitoring device attached to the subject by the hierarchical control architecture system,
- wherein the subject's hemodynamic data is received by the logic-based controller and the at least one adaptive controller,
- wherein the at least one adaptive controller and the logic-based controller are in communication with one another;
c) generating by the at least one adaptive controller an altered infusion rate based on the subject's hemodynamic data, previous infusion rates, and internal parameters and states of the at least one adaptive controller;
d) verifying by the logic-based controller that the subject's hemodynamic data and the at least one adaptive controller states do not violate rules governing the operation of the at least one adaptive controller;
e) sending the altered infusion rate from the logic-based controller to the at least one infusion pump;
f) automatically administering by the at least one infusion pump the fluid and/or cardiovascular drug to the subject at the altered infusion rate or infusion rates upon receipt of the altered infusion rate or infusion rates; and
g) repeating steps b) - f) at set time intervals.

2. The system of claim 1, wherein the at least one adaptive controller uses a function approximator to identify dynamics and parameters of the subject based on sensor data and previous infusion rates, preferably wherein the function approximator is a neural network.

3. The system of claim 1 or 2, wherein administering the fluid restores blood volume in the circulatory system of the subject to an acceptable level.

4. The system of claim 3, wherein the restoration of blood volume in the circulatory system of the subject is determined by the subject's heart rate, mean arterial pressure, stroke volume variation, pulse pressure variation, dynamic arterial elastance, urine output rate, central venous pressure, pleth variability index, systolic pressure variation, systolic pressure, or diastolic pressure.

5. The system of any one of claims 1 to 4, wherein the cardiovascular drug improves the subject's cardiovascular or hemodynamic state to a clinically- acceptable level, preferably wherein the subject's cardiovascular or hemodynamic state is measured using the subject's heart rate, mean arterial pressure, urine output rate, central venous pressure, systolic pressure, diastolic pressure, cardiac output, cardiac index, systemic vascular resistance, or stroke volume..

6. The system of any one of claims 1 to 5, wherein the hemodynamic sensor data comprises the subject's blood pressure, heart rate, stroke volume variation, pleth variability index, urine output rate, central venous pressure, pulse pressure variation, dynamic arterial elastance, systolic pressure variation, mean arterial pressure, systolic pressure, diastolic pressure, cardiac output, cardiac index, systemic vascular resistance, or stroke volume.

7. The system of claim 6, wherein the hemodynamic sensor data is measured invasively or non-invasively.

8. The system of any one of claims 1 to 7, wherein the logic-based controller disengages the at least one adaptive controller if at least one performance criterion is violated.

9. The system of claim 8, wherein the at least one performance criterion comprises changes to a function approximator parameter or a sustained increase in infusion rate without improvements in subject's fluid resuscitation, hemodynamic, or cardiovascular status as measured by hemodynamic sensor data.

10. The system of any one of claims 1 to 9, wherein the logic-based controller is a rule-based expert system.

11. The system of any one of claims 1 to 10, further comprising:
g) monitoring the subject's hemodynamic sensor data after receiving the fluid and/or cardiovascular drug at the altered infusion rate;
h) obtaining the subject's hemodynamic sensor data after receiving the fluid and/or cardiovascular drug at the altered infusion rate; and
i) sending the subject's hemodynamic sensor data obtained after the subject received the fluid and/or cardiovascular drug or at the altered infusion rate to the hierarchical control architecture system.

12. The system of any one of claims 1 to 11, wherein the adaptive controller is designed by assuming that fluid and/or cardiovascular drug distribution in the body is governed by a compartmental dynamical system.

13. The system of claim 12, wherein the hierarchical control architecture system comprises closed-loop system dynamics, wherein a fictitious state is added to the closed-loop system dynamics, wherein the value of the fictitious state rate of change at each instant in time is equal to the sum of the value of the fictitious state at that time instant multiplied by a scaling factor and the blood volume in circulation or cardiovascular drug mass in circulation at that time instant multiplied by a scaling factor, preferably wherein the adaptive controller further comprises an estimator to estimate deviation of the fictitious state and the blood volume in circulation, or fictitious state and a cardiovascular drug mass in circulation from steady state values.

14. The system of any one of claims 1 to 13, wherein the fluid is a crystalloid, colloid, or a blood product, and the cardiovascular drug is a vasoactive drug or an inotropic drug used to improve cardiovascular function.

15. The system of any one of claims 1 to 14, wherein the method administers the fluid and the cardiovascular drug, wherein the hierarchical control architecture system comprises a first adaptive controller and a second adaptive controller, wherein the first adaptive controller is initially engaged by the user through the logic-based controller, wherein the logic-based controller engages the second adaptive controller when at least one performance criterion is met, preferably wherein the performance criterion comprises a user command or inadequate change in hemodynamic measurements while administering fluid or cardiovascular drug..

16. The system of claim 1, wherein the fluid or cardiovascular drug infusion rate is computed using the adaptive controller through the following steps:
a) obtaining sensor data from a sensor measuring
- for fluid administration: stroke volume variation, pulse pressure variation, mean arterial pressure, dynamic arterial elastance, urine output rate, pleth variability index, dynamic arterial elastance, central venous pressure, systolic pressure, diastolic pressure, or systolic pressure variation; or
- for cardiovascular drug administration: mean arterial pressure, cardiac output, cardiac index, heart rate, systolic pressure, diastolic pressure, systemic vascular resistance, or central venous pressure,
wherein the sensor values are recorded and smoothed using a noise reduction technique and wherein measurements are performed invasively or noninvasively;
b) the at least one adaptive controller assumes that a patient is modeled by an augmented dynamical system model consisting of a 2-, 3-, or n-compartment model, characterizing fluid or cardiovascular drug distribution, as well as a fictitious state, wherein the fictitious state follows the same trend as of the fluid volume in circulation in the case of fluid management or cardiovascular drug mass in circulation in the case of cardiovascular drug management with some time lag;
c) a dynamic observer is used to estimate deviation of the fictitious state and the fluid volume in circulation in the case of fluid management or cardiovascular drug mass in circulation in the case of cardiovascular drug management from steady state values;
d) a function approximator is used to approximate the unknown dynamics of the patient and the parameters of the patient, which are modeled by an augmented dynamical system; preferably wherein the function approximator is a neural network;
e) the function approximator weights, sensor measurement values, and infusion rate computed by the adaptive controller at time *t - Dt* or at times *t - Dt* and *t - 2Dt* are used to compute a new infusion rate;
f) the new infusion rate is sent to higher level controller for further processing;
g) the function approximator weights are updated using estimates provided by the dynamic observer, sensor measurement values, and the infusion rate computed by the adaptive controller at time *t - Dt* or at times *t - Dt* and *t - 2Dt;*
h) a delay of T seconds/minutes is introduced; and
i) step c) is repeated to close the loop.

## Patentansprüche

1. System zur Flüssigkeitswiederbelebung und/oder Verabreichung kardiovaskulärer Arzneimittel, wobei das System:
(i) mindestens eine medizinische Überwachungsvorrichtung und mindestens eine Infusionspumpe umfasst und
**dadurch gekennzeichnet ist, dass**
es ein hierarchisches Steuerungsarchitektursystem umfasst, wobei das hierarchische Steuerungsarchitektursystem mindestens eine adaptive Steuereinheit als Steuereinheit der unteren Ebene und eine logikbasierte Steuereinheit als Steuereinheit der höheren Ebene umfasst; und
(ii) zur Durchführung eines Verfahrens konfiguriert ist, umfassend:
a) Einleiten einer Infusion einer Flüssigkeit und/oder eines kardiovaskulären Arzneimittels in ein Subjekt mit einer Infusionsrate;
b) Empfangen hämodynamischer Sensordaten des Subjekts von der mindestens einen an dem Subjekt angebrachten medizinischen Überwachungsvorrichtung durch das hierarchische Steuerungsarchitektursystem,
- wobei die hämodynamischen Daten des Subjekts von der logikbasierten Steuereinheit und der mindestens einen adaptiven Steuereinheit empfangen werden,
- wobei die mindestens eine adaptive Steuereinheit und die logikbasierte Steuereinheit miteinander in Verbindung stehen;
c) Erzeugen einer geänderten Infusionsrate durch die mindestens eine adaptive Steuereinheit auf der Grundlage der hämodynamischen Daten des Subjekts, früherer Infusionsraten und interner Parameter und Zustände der mindestens einen adaptiven Steuereinheit;
d) Überprüfen durch die logikbasierte Steuereinheit, dass die hämodynamischen Daten des Subjekts und die Zustände der mindestens einen adaptiven Steuereinheit keine Regeln verletzen, die den Betrieb der mindestens einen adaptiven Steuereinheit regeln;
e) Senden der geänderten Infusionsrate von der logikbasierten Steuereinheit an die mindestens eine Infusionspumpe;
f) automatisches Verabreichen der Flüssigkeit und/oder des kardiovaskulären Arzneimittels an das Subjekt durch die mindestens eine Infusionspumpe mit der geänderten Infusionsrate oder den geänderten Infusionsraten nach Empfang der geänderten Infusionsrate oder den geänderten Infusionsraten; und
g) Wiederholen der Schritte b) bis f) in bestimmten Zeitabständen.

2. System nach Anspruch 1, wobei die mindestens eine adaptive Steuereinheit einen Funktionsapproximator verwendet, um die Dynamik und die Parameter des Subjekts auf der Grundlage von Sensordaten und früheren Infusionsraten zu identifizieren, wobei der Funktionsapproximator vorzugsweise ein neuronales Netz ist.

3. System nach Anspruch 1 oder 2, wobei die Verabreichung der Flüssigkeit das Blutvolumen im Kreislaufsystem des Subjekts auf ein akzeptables Niveau wiederherstellt.

4. System nach Anspruch 3, wobei die Wiederherstellung des Blutvolumens im Kreislaufsystem des Subjekts durch die Herzfrequenz des Subjekts, den mittleren arteriellen Druck, die Veränderung des Schlagvolumens, die Veränderung des Pulsdrucks, die dynamische arterielle Elastizität, die Urinausscheidungsrate, den zentralvenösen Druck, den Pleth-Variabilitätsindex, die Veränderung des systolischen Drucks, den systolischen Druck oder den diastolischen Druck bestimmt wird.

5. System nach einem der Ansprüche 1 bis 4, wobei das kardiovaskuläre Arzneimittel den kardiovaskulären oder hämodynamischen Zustand des Subjekts auf ein klinisch akzeptables Niveau verbessert, wobei der kardiovaskuläre oder hämodynamische Zustand des Subjekts vorzugsweise unter Verwendung der Herzfrequenz, des mittleren arteriellen Drucks, der Urinausscheidungsrate, des zentralvenösen Drucks, des systolischen Drucks, des diastolischen Drucks, des Herzzeitvolumens, des Herzindex, des systemischen Gefäßwiderstands oder des Schlagvolumens des Subjekts gemessen wird.

6. System nach einem der Ansprüche 1 bis 5, wobei die hämodynamischen Sensordaten den Blutdruck, die Herzfrequenz, die Veränderung des Schlagvolumens, den Pleth-Variabilitätsindex, die Urinausscheidungsrate, den zentralvenösen Druck, die Veränderung des Pulsdrucks, die dynamische arterielle Elastizität, die Veränderung des systolischen Drucks, den mittleren arteriellen Druck, den systolischen Druck, den diastolischen Druck, das Herzzeitvolumen, den Herzindex, den systemischen Gefäßwiderstand oder das Schlagvolumen des Subjekts umfassen.

7. System nach Anspruch 6, wobei die hämodynamischen Sensordaten invasiv oder nicht-invasiv gemessen werden.

8. System nach einem der Ansprüche 1 bis 7, wobei die logikbasierte Steuereinheit die mindestens eine adaptive Steuereinheit deaktiviert, wenn mindestens ein Leistungskriterium verletzt wird.

9. System nach Anspruch 8, wobei das mindestens eine Leistungskriterium Änderungen eines Parameters eines Funktionsapproximators oder eine anhaltende Erhöhung der Infusionsrate ohne Verbesserung der Flüssigkeitswiederbelebung, des hämodynamischen oder des kardiovaskulären Status des Subjekts, gemessen durch hämodynamische Sensordaten, umfasst.

10. System nach einem der Ansprüche 1 bis 9, wobei die logikbasierte Steuereinheit ein regelbasiertes Expertensystem ist.

11. System nach einem der Ansprüche 1 bis 10, ferner umfassend:
g) Überwachen der hämodynamischen Sensordaten des Subjekts nach Erhalt der Flüssigkeit und/oder des kardiovaskulären Arzneimittels mit der geänderten Infusionsrate;
h) Erfassen der hämodynamischen Sensordaten des Subjekts nach Erhalt der Flüssigkeit und/oder des kardiovaskulären Arzneimittels mit der geänderten Infusionsrate; und
i) Senden der hämodynamischen Sensordaten des Subjekts, die erhalten wurden, nachdem das Subjekt die Flüssigkeit und/oder das kardiovaskuläre Arzneimittel oder mit der geänderten Infusionsrate erhalten hat, an das hierarchische Steuerungsarchitektursystem.

12. System nach einem der Ansprüche 1 bis 11, wobei die adaptive Steuereinheit unter der Annahme entworfen wird, dass die Flüssigkeits- und/oder kardiovaskuläre Arzneimittelverteilung im Körper durch ein kompartimentäres dynamisches System geregelt wird.

13. System nach Anspruch 12, wobei das hierarchische Steuerungsarchitektursystem eine Systemdynamik mit geschlossenem Regelkreis umfasst, wobei ein fiktiver Zustand zur Dynamik des geschlossenen Regelkreises hinzugefügt wird, wobei der Wert der Änderungsrate des fiktiven Zustands zu jedem Zeitpunkt gleich der Summe des Werts des fiktiven Zustands zu diesem Zeitpunkt, multipliziert mit einem Skalierungsfaktor, und des Blutvolumens im Kreislauf oder der kardiovaskulären Arzneimittelmasse im Kreislauf zu diesem Zeitpunkt, multipliziert mit einem Skalierungsfaktor, ist, wobei die adaptive Steuereinheit vorzugsweise ferner einen Schätzer umfasst, um die Abweichung des fiktiven Zustands und des Blutvolumens im Kreislauf oder des fiktiven Zustands und einer kardiovaskulären Arzneimittelmasse im Kreislauf von den Werten des stationären Zustands zu schätzen.

14. System nach einem der Ansprüche 1 bis 13, wobei die Flüssigkeit ein Kristalloid, ein Kolloid oder ein Blutprodukt ist und das kardiovaskuläre Arzneimittel ein vasoaktives Arzneimittel oder ein inotropes Arzneimittel ist, das zur Verbesserung der kardiovaskulären Funktion verwendet wird.

15. System nach einem der Ansprüche 1 bis 14, wobei das Verfahren die Flüssigkeit und das kardiovaskuläre Arzneimittel verabreicht, wobei das hierarchische Steuerungsarchitektursystem eine erste adaptive Steuereinheit und eine zweite adaptive Steuereinheit umfasst, wobei die erste adaptive Steuereinheit anfänglich durch den Benutzer über die logikbasierte Steuereinheit aktiviert wird, wobei die logikbasierte Steuereinheit die zweite adaptive Steuereinheit aktiviert, wenn mindestens ein Leistungskriterium erfüllt ist, wobei das Leistungskriterium vorzugsweise einen Benutzerbefehl oder eine unzureichende Änderung der hämodynamischen Messungen während der Verabreichung von Flüssigkeit oder kardiovaskulärem Arzneimittel umfasst.

16. System nach Anspruch 1, wobei die Infusionsrate der Flüssigkeit oder des kardiovaskulären Arzneimittels unter Verwendung der adaptiven Steuereinheit durch die folgenden Schritte berechnet wird:
a) Erfassen von Sensordaten von einem Sensor, der Folgendes misst:
- für die Verabreichung von Flüssigkeiten: die Veränderung des Schlagvolumens, die Veränderung des Pulsdrucks, den mittleren arteriellen Druck, die dynamische arterielle Elastizität, die Urinausscheidungsrate, den Pleth-Variabilitätsindex, die dynamische arterielle Elastizität, den zentralvenösen Druck, den systolischen Druck, den diastolischen Druck oder die Veränderung des systolischen Drucks; oder
- für die Verabreichung kardiovaskulärer Arzneimittel: den mittleren arteriellen Druck, das Herzzeitvolumen, den Herzindex, die Herzfrequenz, den systolischen Druck, den diastolischen Druck, den systemischen Gefäßwiderstand oder den zentralen Venendruck,
wobei die Sensorwerte aufgezeichnet und mit Hilfe einer Rauschunterdrückungstechnik geglättet werden und wobei die Messungen invasiv oder nichtinvasiv durchgeführt werden;
b) die mindestens eine adaptive Steuereinheit geht davon aus, dass ein Patient durch ein erweitertes dynamisches Systemmodell modelliert wird, das aus einem 2-, 3- oder n-Kompartiment-Modell besteht, das die Flüssigkeits- oder kardiovaskuläre Arzneimittelverteilung kennzeichnet, sowie einem fiktiven Zustand, wobei der fiktive Zustand mit einer gewissen Zeitverzögerung dem gleichen Trend folgt wie das Flüssigkeitsvolumen im Kreislauf im Falle des Flüssigkeitsmanagements oder die kardiovaskuläre Arzneimittelmasse im Kreislauf im Falle des kardiovaskulären Arzneimittelmanagements;
c) ein dynamischer Beobachter wird verwendet, um die Abweichung des fiktiven Zustands und des Flüssigkeitsvolumens im Kreislauf im Falle eines Flüssigkeitsmanagements oder der kardiovaskulären Arzneimittelmasse im Kreislauf im Falle eines kardiovaskulären Arzneimittelmanagements von den Werten des stationären Zustands zu schätzen;
d) ein Funktionsapproximator wird verwendet, um die unbekannte Dynamik des Patienten und die Parameter des Patienten, die durch ein erweitertes dynamisches System modelliert werden, zu approximieren; wobei vorzugsweise der Funktionsapproximator ein neuronales Netz ist;
e) die von der adaptiven Steuereinheit zum Zeitpunkt *t - Dt* oder zu den Zeitpunkten *t - Dt* und *t - 2Dt* berechneten Funktionsapproximator-Gewichte, Sensormesswerte und Infusionsrate werden zur Berechnung einer neuen Infusionsrate verwendet;
f) die neue Infusionsrate wird zur weiteren Verarbeitung an die übergeordnete Steuereinheit gesendet;
g) die Funktionsapproximator-Gewichte werden unter Verwendung der vom dynamischen Beobachter gelieferten Schätzungen, der Sensormesswerte und der Infusionsrate, die von der adaptiven Steuereinheit zum Zeitpunkt *t - Dt* oder zu den Zeitpunkten *t - Dt* und *t - 2Dt* berechnet wurde, aktualisiert;
h) Einführen einer Verzögerung von *T* Sekunden/Minuten; und
i) Schritt c) wird wiederholt, um die Schleife zu schließen.

## Revendications

1. Un système de réanimation par fluide et/ou d'administration de médicament cardiovasculaire, dans lequel le système :
(i) comprend au moins un dispositif de surveillance médicale, au moins une pompe à perfusion et
**caractérisé en ce que**
il comprend un système d'architecture de commande hiérarchique, dans lequel le système d'architecture de commande hiérarchique comprend au moins un régulateur adaptatif en tant que régulateur de niveau inférieur et un régulateur basé sur la logique en tant que régulateur de niveau supérieur ; et
(ii) est configuré pour réaliser un procédé comprenant :
a) initier une perfusion d'un fluide et/ou d'un médicament cardiovasculaire dans un X sujet à un débit de perfusion ;
b) recevoir des données de capteur hémodynamiques du sujet provenant d'au moins un dispositif de surveillance médicale attaché au sujet par le système d'architecture de commande hiérarchique,
- dans lequel les données hémodynamiques du sujet sont reçues par le régulateur basé sur la logique et au moins un régulateur adaptatif,
- dans lequel au moins un régulateur adaptatif et le régulateur basé sur la logique sont en communication l'un avec l'autre ;
c) générer, par au moins un régulateur adaptatif, un débit de perfusion modifié basé sur les données hémodynamiques du sujet, les débits de perfusion précédents, et les paramètres internes et états d'au moins un régulateur adaptatif ;
d) vérifier, par le régulateur basé sur la logique, que les données hémodynamiques du sujet et les états d'au moins un régulateur adaptatif ne violent pas les règles régissant le fonctionnement d'au moins un régulateur adaptatif ;
e) envoyer le débit de perfusion modifié du régulateur basé sur la logique à au moins une pompe à perfusion ;
f) administrer automatiquement, par au moins une pompe à perfusion, le fluide et/ou le médicament cardiovasculaire au sujet au débit de perfusion modifié ou aux débits de perfusion modifiés lors de la réception du débit de perfusion modifié ou des débits de perfusion modifiés ; et
g) répéter les étapes b) à f) à des intervalles de temps définis.

2. Le système selon la revendication 1, dans lequel au moins un régulateur adaptatif utilise un approximant de fonction pour identifier la dynamique et les paramètres du sujet sur la base des données de capteur et des débits de perfusion précédents, de préférence dans lequel l'approximant de fonction est un réseau de neurones.

3. Le système selon la revendication 1 ou 2, dans lequel l'administration du fluide restaure le volume sanguin dans le système circulatoire du sujet à un niveau cliniquement acceptable.

4. Le système selon la revendication 3, dans lequel la restauration du volume sanguin dans le système circulatoire du sujet est déterminée par la fréquence cardiaque du sujet, la pression artérielle moyenne, la variation du volume d'éjection systolique, la variation de la pression pulsée, l'élasticité artérielle dynamique, le débit urinaire, la pression veineuse centrale, l'indice de variabilité pléthysmographique, la variation de la pression systolique, la pression systolique ou la pression diastolique.

5. Le système selon l'une quelconque des revendications 1 à 4, dans lequel le médicament cardiovasculaire améliore l'état cardiovasculaire ou hémodynamique du sujet à un niveau cliniquement acceptable, de préférence lequel l'état cardiovasculaire ou hémodynamique du sujet est mesuré à l'aide de la fréquence cardiaque du sujet, la pression artérielle moyenne, le débit urinaire, la pression veineuse centrale, la pression systolique, la pression diastolique, le débit cardiaque, l'index cardiaque, la résistance vasculaire systémique ou le volume d'éjection systolique.

6. Le système selon l'une quelconque des revendications 1 à 5, dans lequel les données de capteur hémodynamiques comprennent la pression sanguine du sujet, la fréquence cardiaque, la variation du volume d'éjection systolique, l'indice de variabilité pléthysmographique, le débit urinaire, la pression veineuse centrale, la variation de la pression pulsée, l'élasticité artérielle dynamique, la variation de la pression systolique, la pression artérielle moyenne, la pression systolique, la pression diastolique, le débit cardiaque, l'index cardiaque, la résistance vasculaire systémique ou le volume d'éjection systolique.

7. Le système selon la revendication 6, dans lequel les données de capteur hémodynamiques sont mesurées de manière invasive ou de manière non invasive.

8. Le système selon l'une quelconque des revendications 1 à 7, dans lequel le régulateur basé sur la logique désengage au moins un régulateur adaptatif si au moins un critère de performance est violé.

9. Le système selon la revendication 8, dans lequel au moins un critère de performance comprend des modifications d'un paramètre d'approximant de fonction ou une augmentation soutenue du débit de perfusion sans amélioration de la réanimation par fluide, de l'état hémodynamique ou de l'état cardiovasculaire du sujet tel que mesuré par les données de capteur hémodynamiques.

10. Le système selon l'une quelconque des revendications 1 à 9, dans lequel le régulateur basé sur la logique est un système expert basé sur des règles.

11. Le système selon l'une quelconque des revendications 1 à 10, comprenant en outre :
g) la surveillance des données de capteur hémodynamiques du sujet après réception du fluide et/ou du médicament cardiovasculaire au débit de perfusion modifié ;
h) l'obtention des données de capteur hémodynamiques du sujet après réception du fluide et/ou du médicament cardiovasculaire au débit de perfusion modifié ; et
i) l'envoi des données de capteur hémodynamiques du sujet obtenues après que le sujet a reçu le fluide et/ou le médicament cardiovasculaire ou au débit de perfusion modifié au système d'architecture de commande hiérarchique.

12. Le système selon l'une quelconque des revendications 1 à 11, dans lequel le régulateur adaptatif est conçu en supposant que la distribution du fluide et/ou du médicament cardiovasculaire dans le corps est régie par un système dynamique compartimental.

13. Le système selon la revendication 12, dans lequel le système d'architecture de commande hiérarchique comprend une dynamique de système en boucle fermée, lequel un état fictif est ajouté à la dynamique de système en boucle fermée, dans lequel la valeur du taux de variation de l'état fictif à chaque instant est égale à la somme de la valeur de l'état fictif à cet instant multipliée par un facteur d'échelle et du volume sanguin en circulation ou de la masse de médicament cardiovasculaire en circulation à cet instant multipliée par un facteur d'échelle, de préférence lequel le régulateur adaptatif comprend en outre un estimateur pour estimer l'écart de l'état fictif et du volume sanguin en circulation, ou de l'état fictif et d'une masse de médicament cardiovasculaire en circulation par rapport aux valeurs d'état stationnaire.

14. Le système selon l'une quelconque des revendications 1 à 13, dans lequel le fluide est un cristalloïde, un colloïde ou un produit sanguin, et lequel le médicament cardiovasculaire est un médicament vasoactif ou un médicament inotrope utilisé pour améliorer la fonction cardiovasculaire.

15. Le système selon l'une quelconque des revendications 1 à 14, dans lequel le procédé administre le fluide et le médicament cardiovasculaire, lequel le système d'architecture de commande hiérarchique comprend un premier régulateur adaptatif et un second régulateur adaptatif, lequel le premier régulateur adaptatif est initialement engagé par l'utilisateur via le régulateur basé sur la logique, lequel le régulateur basé sur la logique engage le second régulateur adaptatif lorsque au moins un critère de performance est satisfait, de préférence lequel le critère de performance comprend une commande utilisateur ou une modification inadéquate des mesures hémodynamiques lors de l'administration du fluide ou du médicament cardiovasculaire.

16. Le système selon la revendication 1, dans lequel le débit de perfusion de fluide ou de médicament cardiovasculaire est calculé à l'aide du régulateur adaptatif selon les étapes suivantes :
a) obtention de données de capteur provenant d'un capteur mesurant
- pour l'administration de fluide : la variation du volume d'éjection systolique, la variation de la pression pulsée, la pression artérielle moyenne, l'élasticité artérielle dynamique, le débit urinaire, l'indice de variabilité pléthysmographique, l'élasticité artérielle dynamique, la pression veineuse centrale, la pression systolique, la pression diastolique ou la variation de la pression systolique ; ou
- pour l'administration de médicament cardiovasculaire : la pression artérielle moyenne, le débit cardiaque, l'index cardiaque, la fréquence cardiaque, la pression systolique, la pression diastolique, la résistance vasculaire systémique ou la pression veineuse centrale,
dans lequel les valeurs du capteur sont enregistrées et lissées à l'aide d'une technique de réduction du bruit et lequel les mesures sont effectuées de manière invasive ou de manière non invasive ;
b) au moins un régulateur adaptatif suppose qu'un patient est modélisé par un modèle dynamique augmenté constitué d'un modèle compartimental à 2, 3 ou n compartiments, caractérisant la distribution du fluide ou du médicament cardiovasculaire, ainsi qu'un état fictif, lequel l'état fictif suit la même tendance que le volume de fluide en circulation dans le cas de la gestion de fluide ou la masse de médicament cardiovasculaire en circulation dans le cas de la gestion de médicament cardiovasculaire avec un certain retard temporel ;
c) un observateur dynamique est utilisé pour estimer l'écart de l'état fictif et du volume de fluide en circulation dans le cas de la gestion de fluide ou de la masse de médicament cardiovasculaire en circulation dans le cas de la gestion de médicament cardiovasculaire par rapport aux valeurs d'état stationnaire ;
d) un approximant de fonction est utilisé pour approximer la dynamique inconnue du patient et les paramètres du patient, lesquels sont modélisés par un modèle dynamique augmenté ; de préférence lequel l'approximant de fonction est un réseau de neurones ;
e) les poids de l'approximant de fonction, les valeurs de mesure du capteur et le débit de perfusion calculé par le régulateur adaptatif à l'instant *t - Dt* ou aux instants *t - Dt* et *t - 2Dt* sont utilisés pour calculer un nouveau débit de perfusion ;
f) le nouveau débit de perfusion est envoyé au régulateur de niveau supérieur pour un traitement ultérieur ;
g) les poids de l'approximant de fonction sont mis à jour à l'aide des estimations fournies par l'observateur dynamique, des valeurs de mesure du capteur et du débit de perfusion calculé par le régulateur adaptatif à l'instant *t - Dt* ou aux instants *t - Dt* et *t - 2Dt* ;
h) un délai de *T* secondes/minutes est introduit ; et
i) l'étape c) est répétée pour boucler la boucle.
